# EUROPEAN PATENT SPECIFICATION

(11) **EP 3 920 895 B1**
(45) Date of publication and mention of the grant of the patent: **18.03.2026**
(21) Application number: 20752141.0
(22) Date of filing: 03.02.2020
(51) Int. Cl.: A61K 31/165, A61K 31/496, A61K 31/275, A61K 31/341, A61K 31/36, A61K 31/4406, A61K 31/495, A61K 31/513, A61K 31/5375, A61K 31/63, A61P 1/00, A61P 39/02, A61K 31/4745, A61P 35/00

(54) **PIPERAZINE DIETHANOL COMPOUND FOR USE IN A METHOD OF PREVENTING OR TREATING CHEMOTHERAPY- OR RADIOTHERAPY-INDUCED GASTROINTESTINAL INJURY**
PIPERAZINDIETHANOL VERBINDUNG ZUR VERWENDING IN DER VORBEUGUNG ODER BEHANDLUNG VON DURCH CHEMOTHERAPIE ODER STRAHLENTHERAPIE VERURSACHTEN GASTROINTESTINALEN VERLETZUNGEN
COMPOSÉ PIPÉRAZINE DIÉTHANOL ET SON UTILISATION DANS LA PRÉVENTION OU LE TRAITEMENT D'UNE LÉSION GASTRO-INTESTINALE INDUITE PAR CHIMIOTHÉRAPIE OU RADIOTHÉRAPIE

(30) Priority: 05.02.2019 US 201962801185 P
(43) Date of publication of application: 15.12.2021
(73) Proprietor: Yu, Jian, San Gabriel, CA 91776 (US); Zhang, Lin, San Gabriel, CA 91776 (US); Leibowitz, Brian, Pittsburgh, PA 15206 (US)
(72) Inventor: Yu, Jian, San Gabriel, CA 91776 (US); Zhang, Lin, San Gabriel, CA 91776 (US); Leibowitz, Brian, Pittsburgh, PA 15206 (US)
(74) Representative: Kilburn & Strode LLP
(86) International application number: PCT/US2020/016302
(87) International publication number: WO 2020/163187

(56) References cited:
- WO-A1-2016/020892
- WO-A1-2017/181585
- US-B2- 7 709 216
- LEIBOWITZ ET AL.: "Targeting p53-dependent stem cell loss for intestinal chemoprotection", SCIENCE TRANSLATIONAL MEDICINE, vol. 10, no. 427, February 2018 (2018-02-01), pages eaam7610 - 11, XP055731224
- MUSTATA ET AL.: "Development of Small-Molecule PUMA Inhibitors for Mitigating Radiation- Induced Cell Death", CURRENT TOPICS IN MEDICINAL CHEMISTRY, vol. 11, no. 3, 2011, pages 281 - 290, XP055731253, DOI: 10.2174/156802611794072641

## Description

### BACKGROUND

### I. Field of the Invention

The present disclosure relates generally to the fields of medicine, oncology, and cancer therapy. More particularly, the disclosure relates to compounds, compositions and use thereof in methods of preventing or treating gastrointestinal injury induced by chemotherapy or radiotherapy.

### II. Description of Related Art

Gastrointestinal (GI) side effects are a major dose-limiting factor in chemotherapy and abdominal radiotherapy and can cause long-term complications in cancer survivors. For example, 5-fluorouracil (5-FU), which is commonly used to treat colorectal cancer patients, has been reported to cause chemotherapy induced diarrhea in 50% of patients. While radiation and most chemotherapeutic agents damage multiple tissues and organ systems, irinotecan hydrochloride (CPT-11), which is commonly used in combination with 5-FU to treat colorectal cancer patients, causes selective GI injury, with severe diarrhea and nausea in more than 50% of patients. This acute toxicity is caused by the accumulation of high concentration of SN-38, the active metabolite of CPT-11, in the intestine, as well as the conversion of nontoxic SN-38 glucuronide back to SN-38 by gut bacteria, followed by intestinal reabsorption. SN-38 is an inhibitor of topoisomerase I, a key enzyme in both DNA replication and RNA transcription, and causes replication stress and DNA damage in proliferating cells. Anti-diarrhea medication can help alleviate CPT-11-induced diarrhea but has a limited effect on reducing long-term GI dysfunction. There is currently no US FDA-approved agent that prevents or treats CPT-11-induced GI injury or complication. Therefore, there is an urgent need to develop new therapeutic agents and methods for preventing or treating GI injury induced by chemotherapy or radiotherapy.

The small-molecule PUMA inhibitor (PUMAi) for use in treating chemotherapy-induced gastrointestinal injury is disclosed in Leibowitz et al.; "Targeting p53-dependent stem cell loss for intestinal chemoprotection", Sci. Transl. Med., vol. 10, eaam7610, no. 427, pages 1-11, 7 February 2018.

### SUMMARY

The present invention is defined in the appended claims. Embodiments and examples, which do not fall within these claims serve to clarity the present invention and are for reference purposes only.

Any references in the description to methods of treatment by therapy refer to the compound of the present invention for use in those methods.

In one aspect, the present disclosure provides a compound capable of preventing or treating treatment-induced gastrointestinal injury, e.g., induced by chemotherapy or radiotherapy. In one embodiment, the compound is selected from the group consisting of:

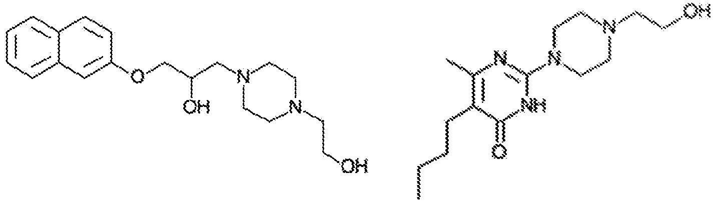

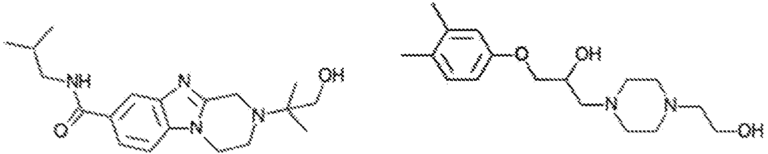

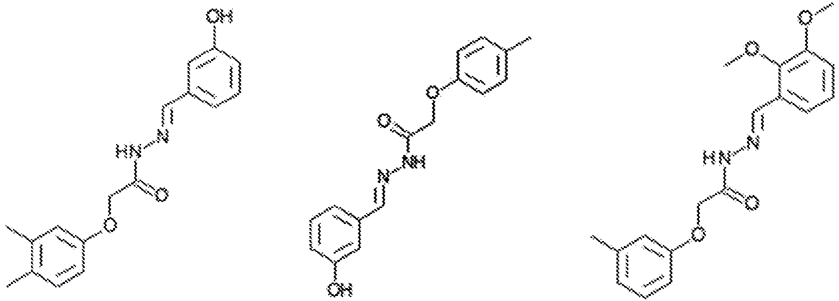

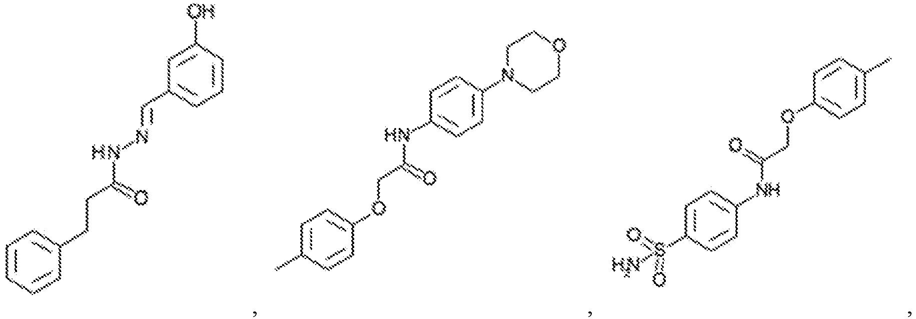

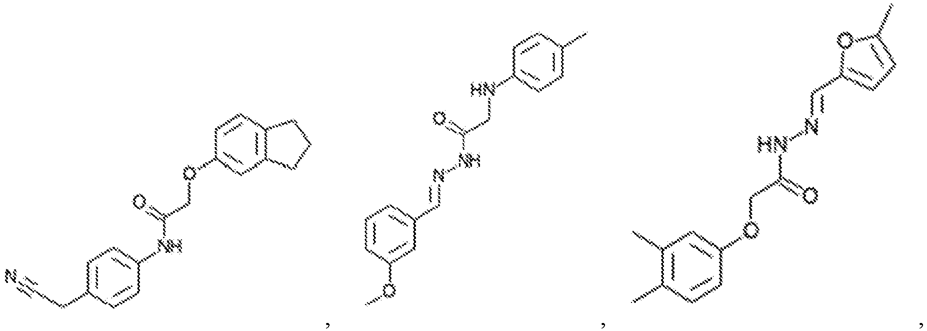

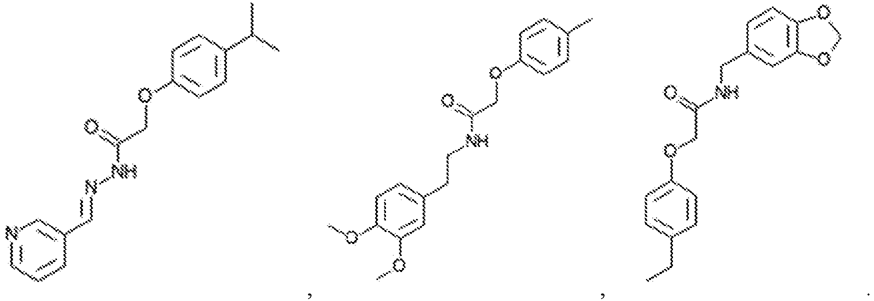

In certain embodiments, the compound is deuterated.

In another aspect, the present disclosure provides a method for preventing or treating treatment-induced gastrointestinal injury. In one embodiment, the method comprises administering to a subject a therapeutically effective amount of the compound described herein, wherein the subject has received, is receiving or will receive a chemotherapy or radiotherapy.

In certain embodiments, the subject has a cancer. In some embodiments, the cancer is a cancer of the skin, breast, pancreas, prostate, ovary, kidney, esophagus, gastrointestinal tract, colon, brain, liver, lung, head and/or neck.

In certain embodiments, administration of the compound inhibits activity of p53 upregulated modulator of apoptosis (PUMA) in at least a non-cancer cell, thereby preventing apoptosis of the non-cancer cell induced by the chemotherapy or radiotherapy. In certain embodiments, the non-cancer cell is a LGR5⁺ stem cell.

In certain embodiments, administration of the compound does not protect cancer cells from the chemotherapy or radiotherapy.

In certain embodiments, the chemotherapy is selected from the group consisting of irinotecan hydrochloride (CPT-11) and 5-fluorouracil (5-FU).

In another aspect, the present disclosure provides a method for treating cancer in a subject. In certain embodiments, the method comprises administering to the subject a chemotherapy or radiotherapy, and administering to the subject a therapeutically effective amount of the compound described herein.

In another aspect, the present disclosure provides a method for screening agents of preventing or treating injury induced by chemotherapy or radiotherapy. In one embodiment, the method comprises: treating an organoid with chemotherapy or radiotherapy; and exposing the organoid with a candidate agent to identify an agent that prevents apoptosis of the organoid.

In certain embodiments, the organoid is derived from a tissue from a human subject. In certain embodiments, the organoid is derived from a gastrointestinal tissue. In certain embodiment, the organoid is a colonic organoid. In certain embodiments, the organoid is derived from a cancer tissue. In certain embodiments, the organoid comprises at least a cancer cell.

In certain embodiments, the agent as identified prevents or treats gastrointestinal injury induced by chemotherapy or radiotherapy *in vivo.*

It is contemplated that any method or composition described herein can be implemented with respect to any other method or composition described herein. Other objects, features and advantages of the present disclosure will become apparent from the following detailed description. It should be understood, however, that the detailed description and the specific examples, while indicating specific embodiments of the invention, are given by way of illustration only, since various changes and modifications will become apparent to those skilled in the art from this detailed description.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

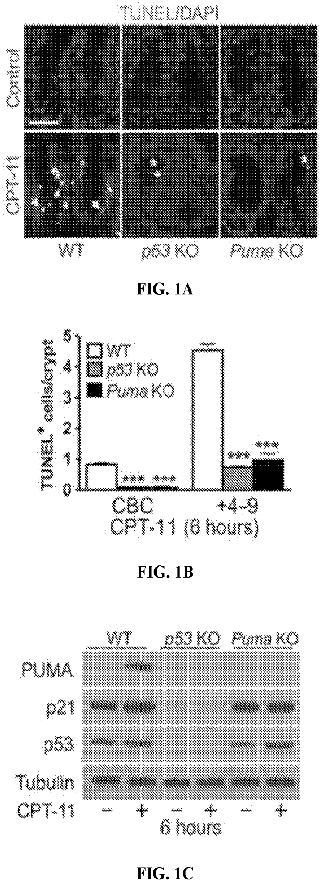

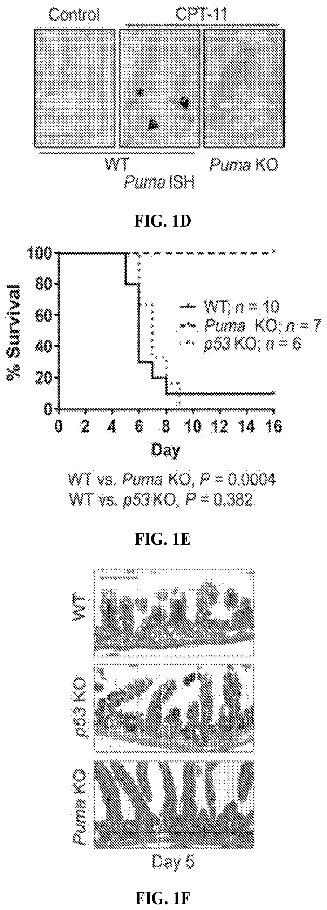

**FIGS. 1A-1F** **show that PUMA mediates CPT-11-induced intestinal injury.** Mice with indicated genotypes were treated with CPT-11 (200 mg/kg) once or as specified and analyzed at indicated times. **(****FIG. 1A****)** Representative images of terminal deoxynucleotidyl transferase-mediated deoxyuridine triphosphate nick end labeling (TUNEL) staining in intestinal crypts at 6 hours. DAPI (4',6-diamidino-2-phenylindole) was used to stain nuclei. Scale bar, 50 um. **(****FIG. 1B****)** Quantitation of TUNEL+ columnar cells at the crypt bottom (CBCs) and +4 to 9 cells in **FIG. 1A. (FIG. 1C****)** Expression of indicated proteins was analyzed by Western blotting at 6 hours. Lysates were pooled from the intestinal mucosa of three mice. Representative results are shown, and similar results were obtained in at least three independent experiments. **(****FIG. 1D****)** Puma RNA in situ hybridization (ISH) in the crypts at 6 hours. Scale bar, 20 um. In **(****FIG. 1A****)** and **(****FIG. 1D****),** arrows indicate CBCs at positions 1 to 3 below Paneth cells, and asterisks indicate +4 to 9 cells at positions 4 to 9 above the CBCs. In **(****FIG. 1A)** to **(FIG. 1D****),** n = 3 mice per group. In **(****FIG. 1B****),** values are means + SEM. ***P < 0.001 (two-tailed Student's t test), knockout (KO) versus wild type (WT). **(****FIG. 1E****)** Survival of mice treated with three consecutive daily doses of CPT-11 (215 mg/kg per day) on days 0, 1, and 2. WT versus Puma KO, P = 0.0004; WT versus p53 KO, P = 0.382 (log-rank test). **(****FIG. 1F****)** Hematoxylin and eosin (H&E) staining of the small intestine from mice on day 5 treated as in **(****FIG. 1E****).** Scale bar, 200 um.

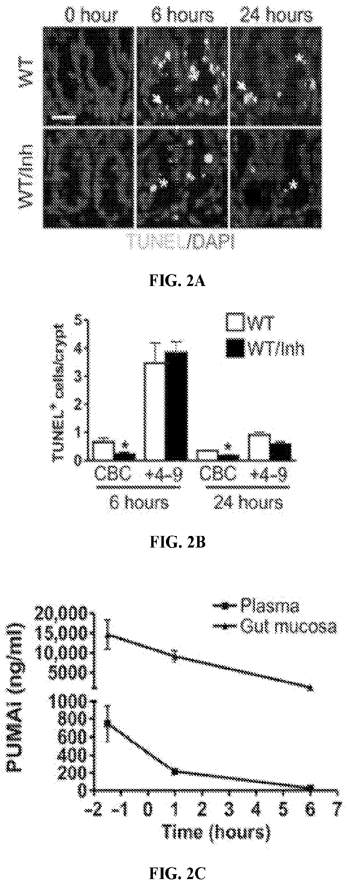

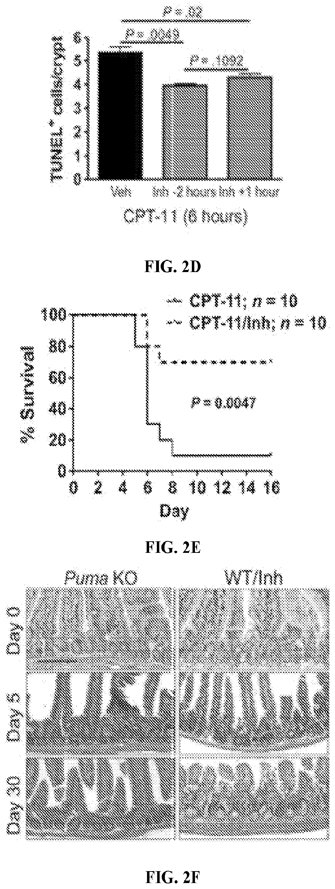

**FIG. 2A-2F** show that PUMAi protects against CPT-11-induced intestinal injury. Mice were treated with CPT-11 with or without PUMA inhibitor (PUMAi). PUMAi (10 mg/kg) or vehicle was given intraperitoneally for 2 hours before CPT-11 or as specified. The small intestine or indicated tissue was analyzed at the indicated times. **(****FIG. 2A****)** Representative images of TUNEL staining in intestinal crypts of mice treated with CPT-11 (200 mg/kg). DAPI was used to stain nuclei. Scale bar, 50 um. Arrows indicate CBCs, and asterisks indicate +4 to 9 cells. **(****FIG. 2B****)** Quantitation of TUNEL+ CBCs and transit-amplifying cells from **(****FIG. 2A****).** Inh, inhibitor. **(****FIG. 2C****)** Tissue distribution of PUMAi at different times after a single injection. **(****FIG. 2D****)** Quantitation of TUNEL+ crypt cells in mice 6 hours after CPT-11 (200 mg/kg) treatment. PUMAi (10 mg/kg) was given 2 hours before or 1 hour after CPT-11 treatment. n = 3 mice per group **(****FIGS. 2B** to **2D****).** In **(****FIG. 2B****)** and **(****FIG. 2D****),** values are means + SEM. *P < 0.05 (twotailed Student's t test). Veh, vehicle. **(****FIG. 2E****)** Survival of WT mice after three doses of CPT-11 (215 mg/kg per day) (done with the control in **FIG. 1E****).** PUMAi (10 mg/kg) was given 2 hours before each dose of CPT-11. P = 0.0047 (log-rank test). **(****FIG. 2F****)** H&E staining of the small intestine from mice treated as in **(****FIG. 2E****)** on days 0, 5 and 30. Scale bar, 200 um.

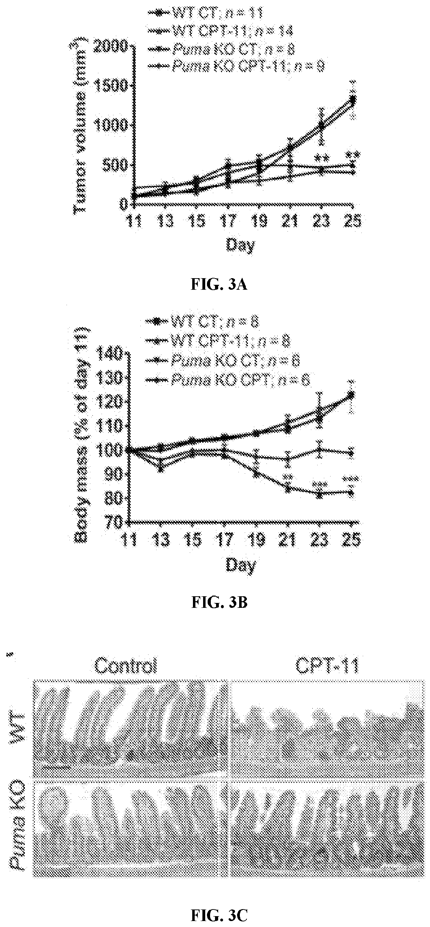

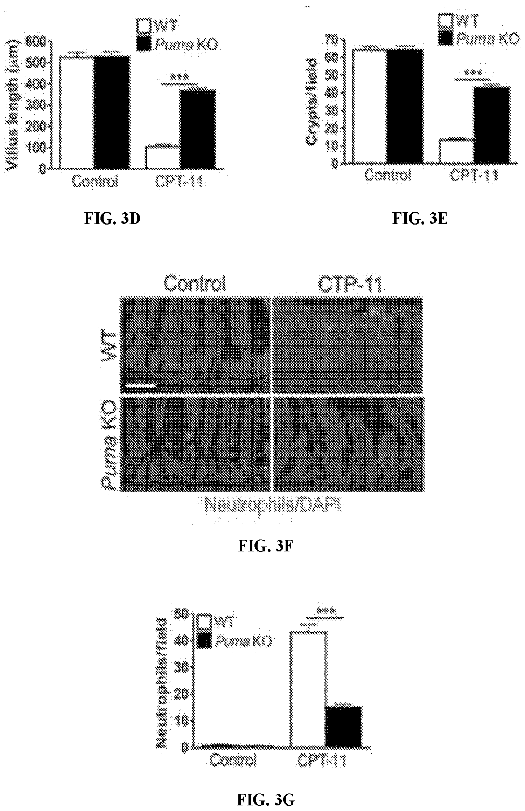

**FIGS. 3A-3G** show that PUMA deficiency protects tumor-bearing mice from chemotherapy-induced GI injury. WT and Puma KO mice bearing Lewis lung carcinoma (LLC) tumors were treated with CPT-11 (200 mg/kg) six times over 15 days, on days 11, 14, 17, 19, 23, and 25. Mice or tumors were analyzed at the indicated times. **(****FIG. 3A****)** Tumor volumes were measured every other day from days 11 to 25. CT, vehicle control. **(****FIG. 3B****)** Body mass was expressed as a percentage of that on day 11 for each mouse from days 11 to 25. **(****FIG. 3C****)** H&E staining of the small intestine tissue on day 25. Scale bar, 100 um. **(****FIG. 3D****)** Villus height from **(****FIG. 3C****).** Measurements were from a minimum of 40 villi per mouse. **(****FIG. 3E****)** Quantitation of intestinal crypts per field from **(****FIG. 3C****). (****FIG. 3F****)** Intestinal neutrophils detected by immunofluorescence on day 25. DAPI was used to stain nuclei. Scale bar, 100 um. **(****FIG. 3G****)** Quantitation of neutrophils per 400Å~ field from the same slides as **(****FIG. 3F****).** In **FIGS. 3A, 3B****,** **3D, 3E,** and **3G****,** values are means + SEM; n = indicated **(****FIGS. 3A** and **3B****)** or 3 to 4 mice per group **(****FIGS. 3D, 3E,** and **3G****).** **P < 0.01 and ***P < 0.001 (two-tailed Student's t test). Puma KO, CPT versus control **(****FIG. 3A****);** CPT-11-treated, Puma KO versus WT **(****FIG. 3B****).**

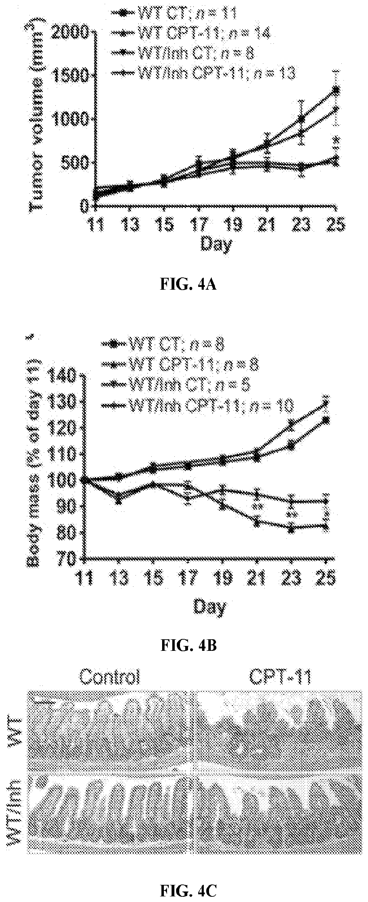

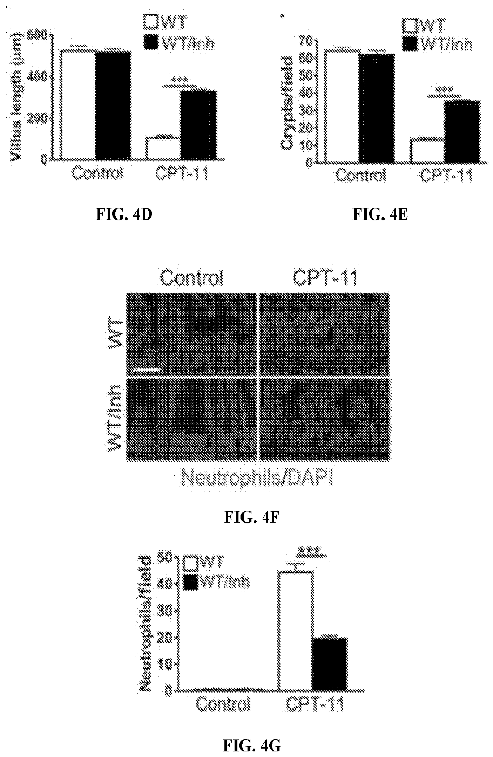

**FIGS. 4A-4G** **show that PUMAi protects tumor-bearing mice from chemotherapy-induced GI injury.** WT mice bearing LLC tumors were treated with CPT-11 (200 mg/kg) six times over 15 days on days 11, 14, 17, 19, 23, and 25. Vehicle or PUMAi was given 2 hours before and 20 hours after each dose of CPT-11. Mice or tumors were analyzed at the indicated times. **(****FIG. 4A****)** Tumor volumes were measured starting on day 11. **(****FIG. 4B****)** Body mass was expressed as a percentage of that on day 11 for each mouse from days 11 to 25. **(****FIG. 4C****)** H&E staining of the small intestine tissue on day 25. Scale bar, 100 um. **(****FIG. 4D****)** Villus height from **(****FIG. 4C****).** Measurements were from a minimum of 40 villi per mouse. (E) Quantitation of intestinal crypts per field from **(****FIG. 4C****). (****FIG. 4F****)** Intestinal neutrophils detected by immunofluorescence on day 25. DAPI was used to stain the nuclei. Scale bar, 100 um. **(****FIG. 4G****)** Quantitation of neutrophils per 400Å~ field from the same slides as **(****FIG. 4F****).** In **FIGS. 4A, 4B****,** **4D, 4E,** and **4G****,** values are means + SEM; n = indicated **(****FIGS. 4A** and **4B****)** or 3 to 4 mice per group **(****FIGS. 4D, 4E,** and **4G****).** *P < 0.05, **P < 0.01, and ***P < 0.001 (twotailed Student's t test). PUMAi, CPT versus control **(****FIG. 4A****);** CPT-11-treated mice, PUMAi versus CT **(****FIG. 4B****).**

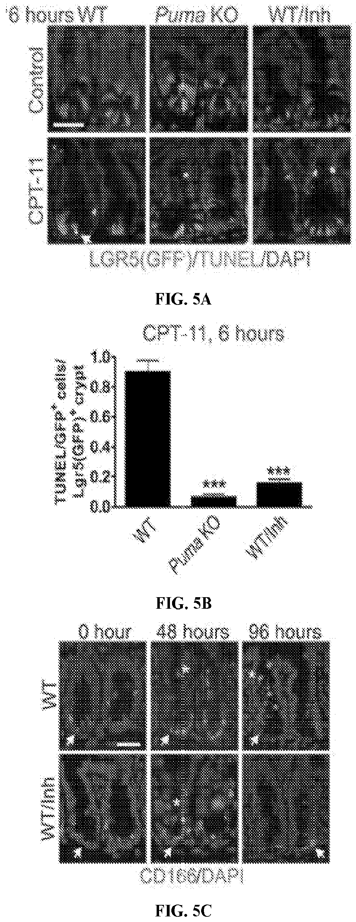

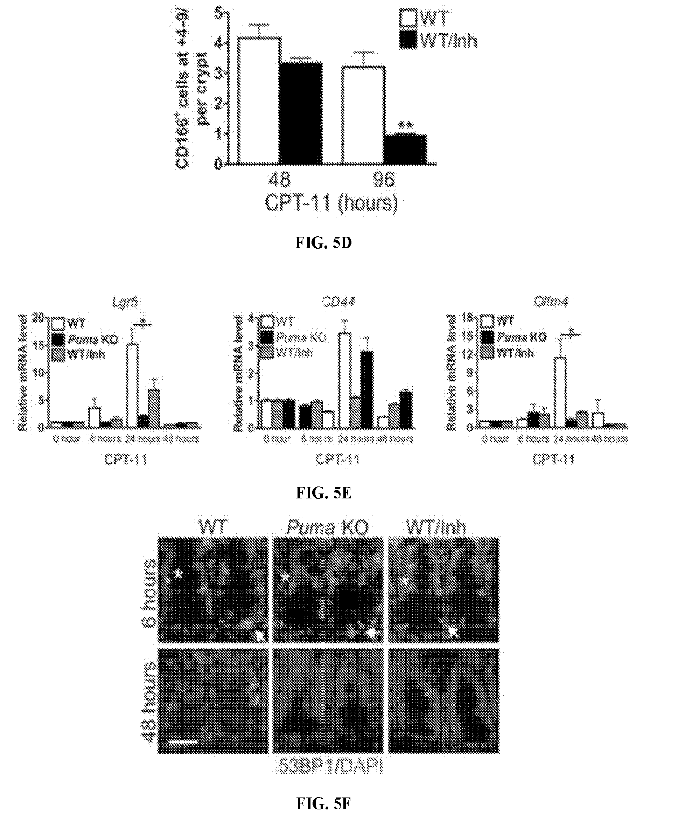

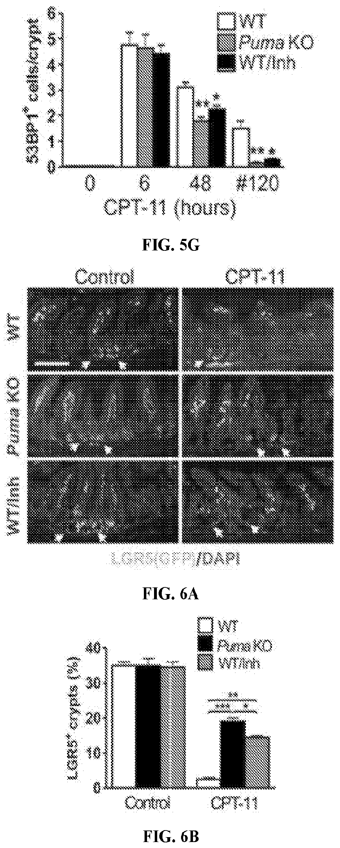

**FIGS. 5A-5G** **show that targeting PUMA protects the LGR5⁺ stem cells** against **CPT-11.** WT and Puma KO mice were treated with CPT-11 (200 mg/kg) and analyzed at the indicated times. PUMAi (10 mg/kg) was given once 2 hours before CPT-11 treatment. **(****FIG. 5A****)** Green fluorescent protein (GFP) (LGR5) and TUNEL immunofluorescence staining in intestinal crypts. Scale bar, 50 m. Arrow indicates double positive cell. **(****FIG. 5B****)** Quantitation of GFP/TUNEL-double-positive cells per GFP-positive crypt from **(****FIG. 5A). (FIG. 5C****)** Immunofluorescence staining for CD166 in the intestinal crypts. Scale bar, 50 um. **(****FIG. 5D**) Quantitation of CD166 cells in the +4 to 9 region in crypts. **(****FIG. 5E**) Indicated mRNAs were analyzed by quantitative reverse transcription polymerase chain reaction (qRT-PCR). Complementary DNAs (cDNAs) were synthesized from RNA pooled from three mice. Values were normalized to Gapdh expression and expressed relative to each gene's own 0-hour control. **(****FIG. 5F**) 53BP1 immunofluorescence staining in intestinal crypts. Scale bar, 50 um. **(****FIG. 5G****)** Quantitation of 53BP1+ crypt cells at 6 and 48 hours after single CPT-11 treatment or #120 hours with three daily doses of CPT-11 (215 mg/kg per day) as in **FIG. 2E****.** In **(****FIG. 5C****)** and **(****FIG. 5F**), arrows indicate CBCs, and asterisks indicate +4 to 9 cells. In **FIGS 5A, 5C**, and **5F**, DAPI was used to stain the nuclei. In **FIGS. 5B****,** **5E****,** and **5G****,** values are means + SEM; n = 3 mice per group. *P < 0.05, **P < 0.01, and ***P < 0.001 [one-way analysis of variance (ANOVA) with Tukey post hoc test performed separately for each time point]. In **(****FIG. 5D**), values are means + SEM; n = 3 mice per group. **P < 0.01 (two-tailed Student's t test).

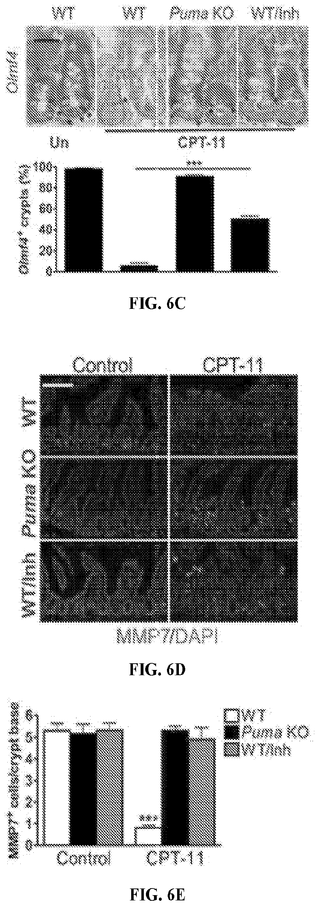

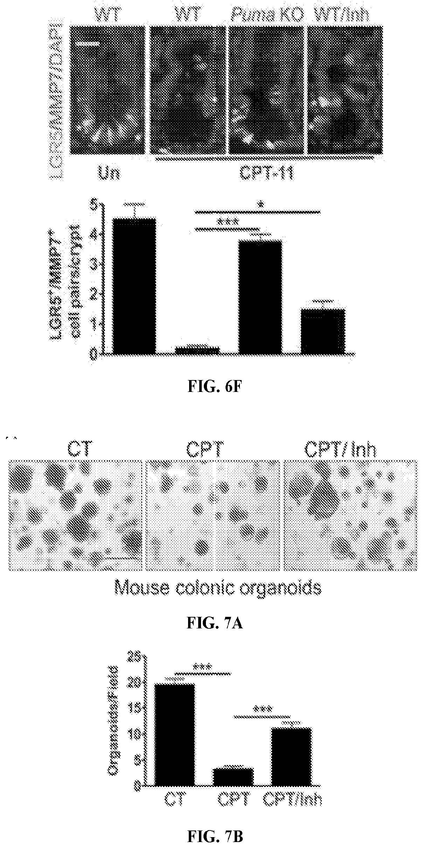

**FIGS. 6A-6F****show that targeting PUMA prevents LGR5⁺ stem cell exhaustion after repeated CPT-11 exposure.** WT and Puma KO mice carrying the Lgr5-EGFP-IRES-creERT2 marking allele were treated with CPT-11 and PUMAi over 2 weeks as in **FIGS. 5A-5G** and analyzed for intestinal phenotypes. **(****FIG. 6A****)** GFP (LGR5) immunofluorescence in intestinal crypts. Arrows indicate LGR5⁺ (GFP) crypts. Scale bar, 100 um. **(****FIG. 6B****)** Quantitation of intestinal crypts containing at least one GFP⁺ (LGR5⁺) cell. **(****FIG. 6C****)** Top: Olfm4 RNA ISH in intestinal crypts. Scale bar, 50 um. Asterisks indicate Olfm4+ crypt cells. Bottom: Percentage of crypts containing at least one Olfm4+ cell. **(****FIG. 6D****)** MMP7 immunofluorescence in intestinal crypts. Scale bar, 100 um. **(****FIG. 6E****)** Quantitation of MMP7+ crypt cells at the crypt base (CBC region). **(****FIG. 6F****)** Top: GFP (LGR5) and MMP7 immunofluorescence in intestinal crypts. Scale bar, 20 um. Asterisks indicate LGRS⁺/MMP7⁺ cell pairs. Bottom: Quantitation of LGR5⁺/MMP7⁺ cell pairs per crypt. In **(****FIG. 6A****), (****FIG. 6D****),** and **(****FIG. 6F****),** DAPI was used to stain nuclei. In **(****FIG. 6B****), (****FIG. 6C****), (****FIG. 6E****),** and **(****FIG. 6F****),** values are means + SEM; n = 3 mice. *P < 0.05, **P < 0.01, and ***P < 0.001 (one-way ANOVA with Tukey post hoc test).

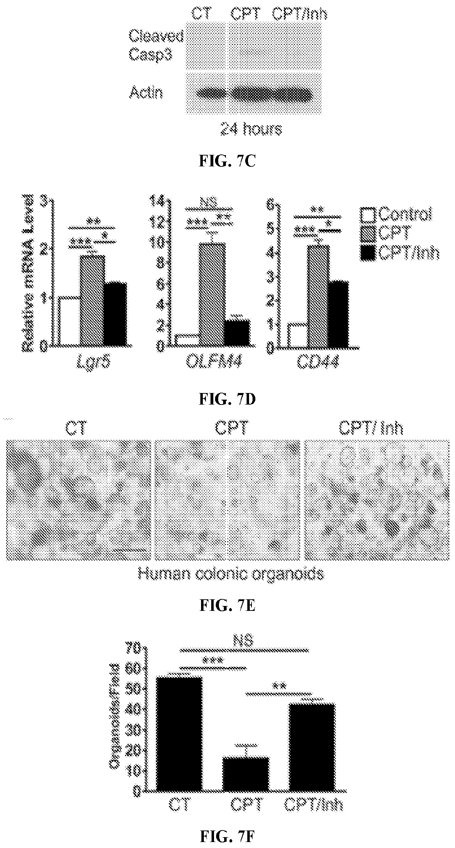

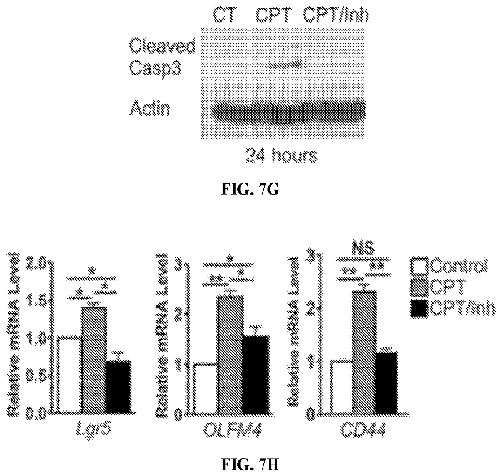

**FIGS. 7A-7H** **show that PUMAi protects mouse and human colonic culture against CPT-induced damage. (****FIG. 7A****)** Representative images of mouse colonic organoids 6 days after CPT treatment. Organoids were treated with vehicle control (CT) or 500 nM CPT (day 1) for 24 hours with or without 50 uM PUMAi and monitored for growth until day 7. Scale bar, 500 um. **(****FIG. 7B****)** Quantitation of organoids 100 um or greater in diameter per field from **(****FIG. 7A****). (****FIG. 7C****)** Western blots for active (cleaved) caspase-3 (Casp3) from organoids 24 hours after CPT treatment. **(****FIG. 7D****)** Indicated mouse mRNAs were analyzed by qRTPCR 24 hours after CPT treatment. NS, not significant. **(****FIG. 7E****)** Representative images of human colonic organoids treated as in **(****FIG. 7A****).** Scale bar, 500 um. **(****FIG. 7F****)** Quantitation of organoids 100 um or greater in diameter per field from **(****FIG. 7E****). (****FIG. 7G****)** Western blots for active (cleaved) caspase-3 from human organoids 24 hours after CPT treatment. **(****FIG. 7H****)** Indicated human mRNAs analyzed 24 hours after CPT treatment. In **(****FIG. 7C****)** and **(****FIG. 7G****),** lysates were prepared from three wells. Actin was used as a control for protein loading. In **(****FIG. 7D****)** and **(****FIG. 7H****),** cDNAs were synthesized from RNA pooled from three cultured wells. Values were normalized to Gapdh and expressed relative to vehicle controls. In **(****FIG. 7B****), (****FIG. 7D****), (****FIG. 7F****),** and **(****FIG. 7H****),** values are means + SEM. *P < 0.05, **P < 0.01, and ***P < 0.001 (one-way ANOVA with Tukey post hoc test).

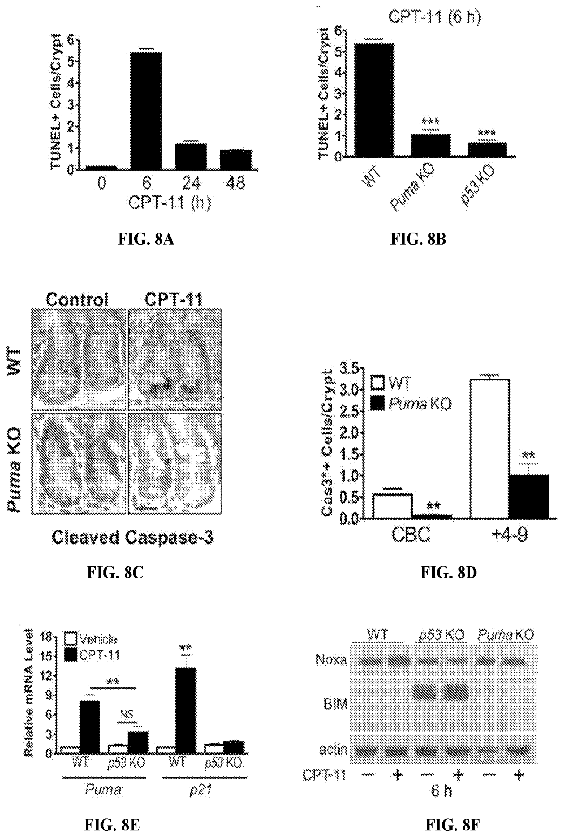

**FIGS. 8A-8F** **show that PUMA KO inhibits CPT-11-induced crypt apoptosis.** Mice were treated with a single dose of CPT-11 (200 mg/kg), and analyzed at indicated times. **(****FIG. 8A****)** Quantitation of TUNEL+ crypt cells in WT mice at indicated times. **(****FIG. 8B****)** Quantitation of TUNEL+ crypt cells at 6 h. **(****FIG. 8C****)** Representative images of active caspase-3 staining in the crypts at 6 h. Bar = 25 µm. (D) Quantitation of active caspase-3+ CBC and +4-9 cells at 6 h. **(****FIG. 8E****)** Indicated mRNAs were analyzed by qRT-PCR. cDNAs were synthesized from RNA pooled from 3 mice. **(****FIG. 8F****)** Indicated proteins at 6 h were analyzed by western blotting. Lysates were pooled from intestinal mucosa from 3 mice. Representative results are shown, and similar results were obtained in at least three independent experiments. **FIGS. 8A, 8B, 8D, 8E****,** values are mean ± SEM, n = 3 mice per group. **P < 0.01; ***P < 0.001 (Student's T Test, two-tailed).

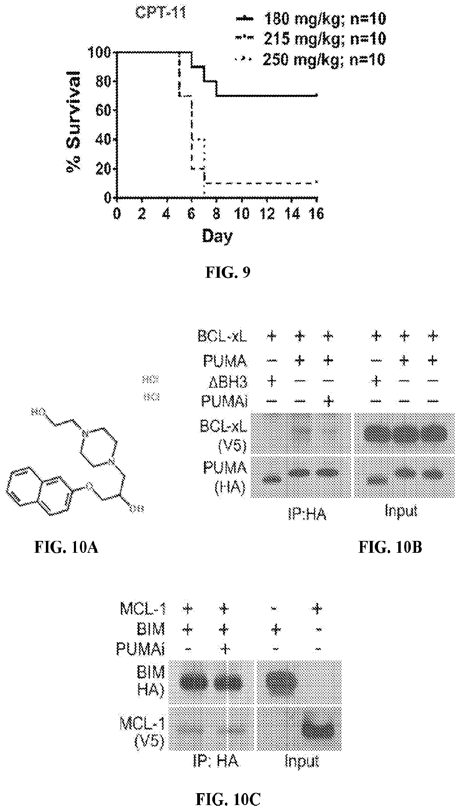

**FIG. 9** **shows that CPT-11 causes dose-dependent lethality in mice.** Survival of WT mice treated with three consecutive daily doses of 180, 215, or 250 mg/kg/day CPT-11 on days 0, 1, and 2. n = 10 mice per group. P values were calculated by log-rank test.

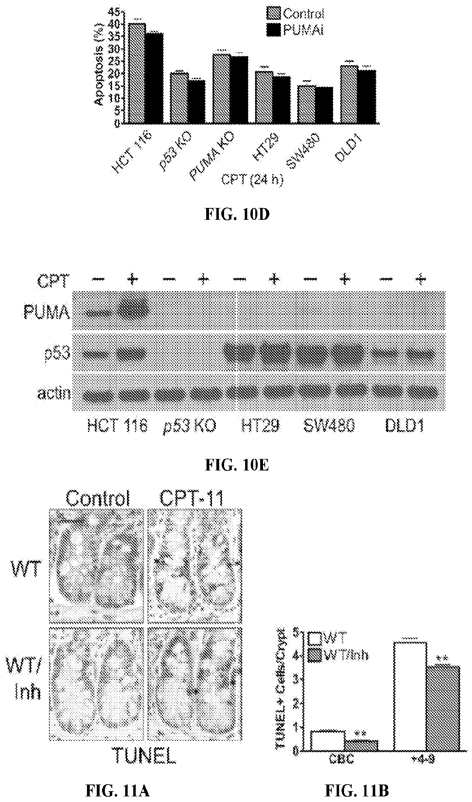

**FIG. 10A-10E** **show that PUMAi does not protect colon cancer cells against CPT-11-induced apoptosis. (****FIG. 10A****)** Structure of the PUMA inhibitor lead compound (PUMAi). **(****FIG. 10B****)** Lysates of 293 cells transfected with individual plasmids for 24 hours were mixed in the indicated combinations. Lysates were incubated with vehicle or PUMA inhibitor (PUMAi, 25 µM) for 15 min before immunoprecipitation with an anti-HA antibody. The HA-bound proteins and input were analyzed by western blotting. **(****FIG. 10C****)** Lysates of 293 cells transfected with individual plasmids for 24 h were mixed in the indicated combinations. Lysates were incubated with vehicle or PUMA Inhibitor (PUMAi, 25 µM) for 15 min before immunoprecipitation with an anti-HA antibody. The HA-bound proteins and input were analyzed by western blotting. **(****FIG. 10D****)** Indicated colon cancer cell lines were treated with camptothecin (CPT, 500 nM) for 24 h. HCT 116 cells and PUMA KO HCT 116 cells have WT p53, others have no (KO) or mutant p53. Apoptosis was measured by nuclear fragmentation assay. Values are mean + SEM; n = 3 independent experiments. **(****FIG. 10E****)** Cells were treated as in D for 24 h and analyzed by western blotting. Representative results are shown, and similar results were obtained in at least three independent experiments.

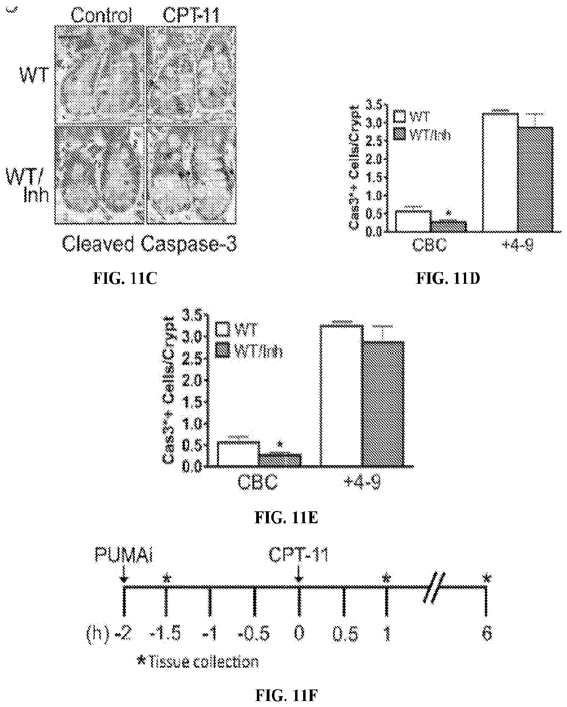

**FIGS. 11A-11F** **show that PUMAi inhibits CPT-11-induced CBC apoptosis.** Mice were treated with a single dose of CPT-11 (200 mg/kg), and analyzed at 6 h. PUMAi was given 2 h before CPT-11. **(****FIG. 11A****)** Representative images of TUNEL staining in intestinal crypts of WT mice with indicated treatment. Bar = 25 µm. **(****FIG. 11B****)** Quantitation of TUNEL+ crypt cells at positions 1-3 (CBC) or 4-9. **(****FIG. 11C****)** Representative images of active caspase-3 staining in intestinal crypts. Bar = 25 µm. **(****FIG. 11D****)** Quantitation of active caspase-3+ crypt cells at positions 1-3 (CBC) or 4-9. **(****FIG. 11E****)** Quantitation of TUNEL+ crypt cells. **(****FIG. 11F****)** Schematic of treatment and tissue collection schedule for PUMAi pharmacokinetic experiment. CPT -11 was administered at 0 h. B, D, and E, values are mean SEM, n = 3 mice per group. A and C, asterisks indicate TA cells. **P < 0.01. *P < 0.05 (Student's T Test, two-tailed).

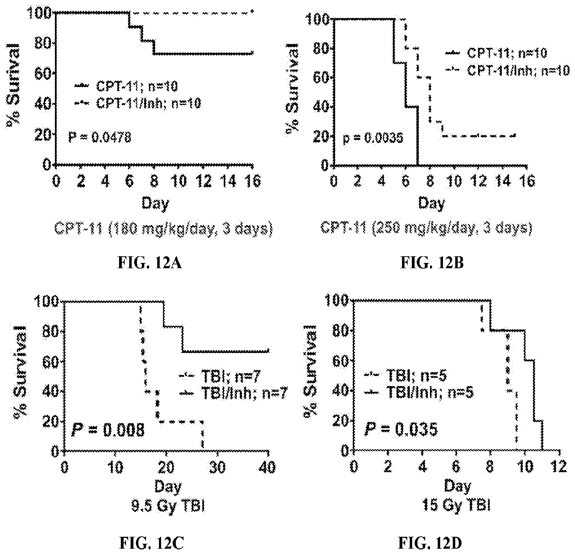

**FIGS. 12A-12D** **show that PUMAi protects against chemotherapy- and radiation-induced lethality. (****FIG. 12A)** and **(FIG. 12B****)** Survival of WT mice treated with 3 daily doses of 180 mg/kg/day (A) and 250 mg/kg/day (B) CPT-11, respectively, on days 0, 1, and 2. PUMAi (10 mg/kg) was given 2 hours before each dose of CPT-11 and 20 hours after the final dose. **(****FIG. 12C)** and **(FIG. 12D****)** Survival of WT mice after total body irradiation (TBI) at 9.5 Gy **(****FIG. 12C****)** and 15 Gy **(****FIG. 12D****).** PUMAi (10 mg/kg) was given 30 min before and 30 min after TBI, and then daily for 4 days. Survivors at 9.5 Gy TBI in C were sacrificed on day 40 with no obvious health problems. P values were calculated by log-rank test.

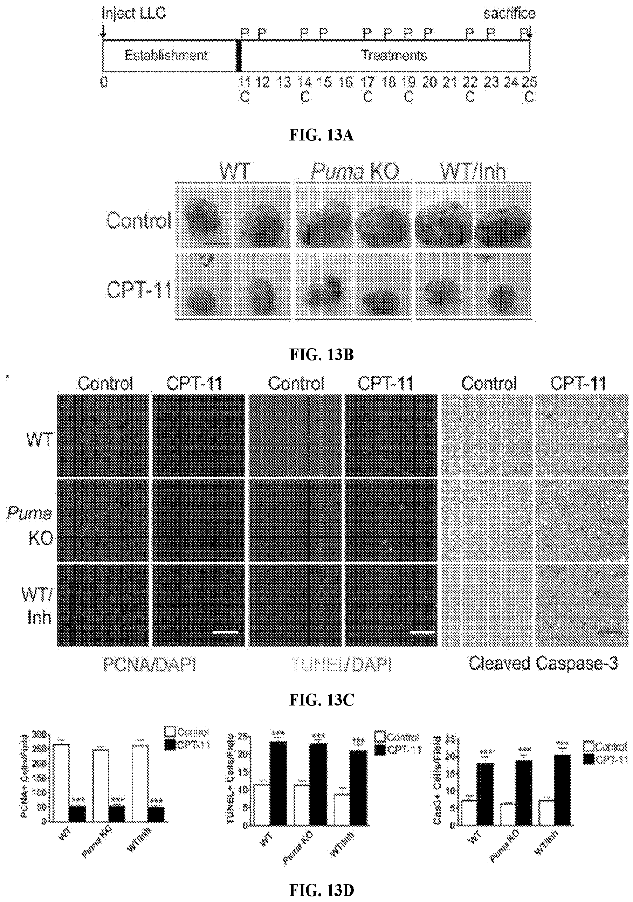

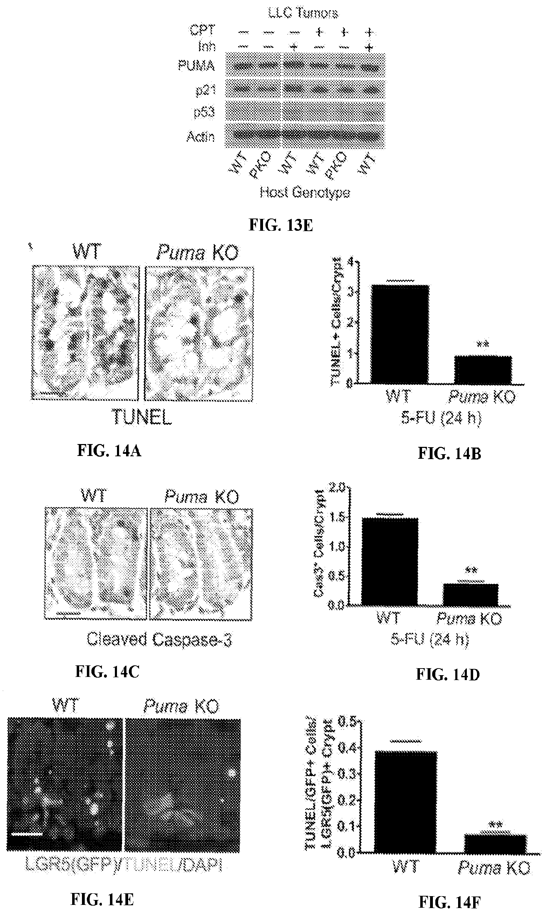

**FIGS. 13A-13E** **show that targeting PUMA does not compromise tumor response to CPT-11. (****FIG. 13A****)** Schematic for tumor establishment and treatments. Mice were injected subcutaneously on their flanks with four million Lewis Lung Carcinoma (LLC) cells. Tumors were allowed to establish for 11 days, and mice were treated with 200 mg/kg CPT-11 (C) 6 times over 2 weeks. PUMAi (P) was given 11 times, 2 hours before and 20 hours after each dose of CPT-11. Mice were sacrificed 4 hours after the last dose of CPT-11. **(****FIG. 13B****)** Representative images of tumors collected after two weeks of treatment. Bar = 1 cm. **(****FIG. 13C****)** Representative images of PCNA, TUNEL, and active (cleaved) caspase-3 staining in LLC tumors after two weeks of CPT-11 treatment. Bar = 100 µm. **(****FIG. 13D****)** Quantitation of PCNA+, TUNEL+, and cleaved caspase-3+ cells per field in C. Values are mean ± SEM, n = 3 tumors per group. **P < 0.01. ***P < 0.001 (Student's T Test, two-tailed). **(****FIG. 13E****)** Western blots of the indicated proteins from tumors treated as in A. Each lane represents a single tumor.
**FIGS. 14A-14F** **show that 5-FU-induced LGR5⁺ cell apoptosis is PUMA-dependent.** WT and Puma KO LGR5- EGFP mice were treated with 200 mg/kg 5-FU for 24 h. **(****FIG. 14A****)** Representative images of TUNEL staining in intestinal crypts. Bar = 25 µm. **(****FIG. 14B****)** Quantitation of TUNEL+ crypt cells. **(****FIG. 14C****)** Representative images of cleaved caspase-3 staining in intestinal crypts. Bar = 25 µm. **(****FIG. 14D****)** Quantitation of active (cleaved) caspase-3+ crypt cells. **(****FIG. 14E****)** Representative images of LGR5 (GFP)/TUNEL double immunofluorescence in intestinal crypts. **(****FIG. 14F****)** Quantitation of GFP/TUNEL double positive cells in GFP-positive crypts. B, D, and F, values are mean ± SEM, n = 3 mice per group. **P < 0.01 (Student's T Test, two-tailed).

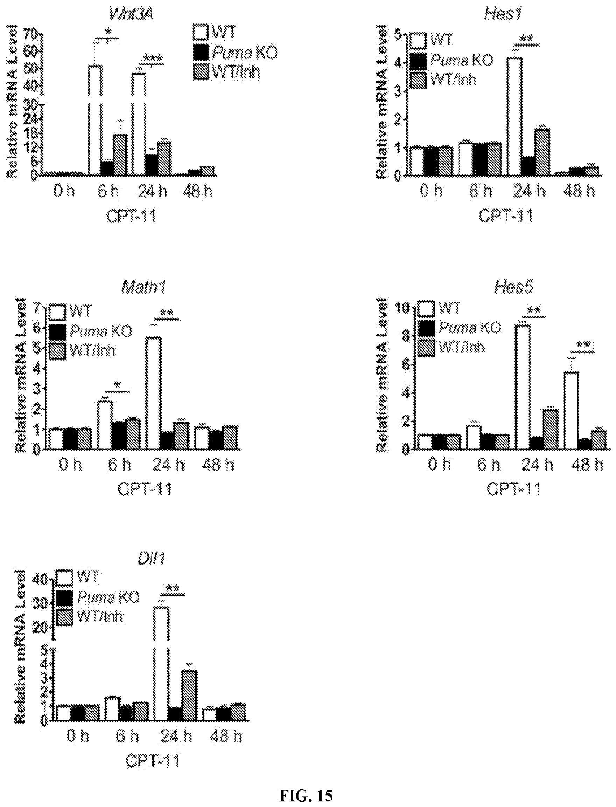

**FIG. 15** **shows that Puma KO and PUMAi suppress CPT-11-induced expression of WNT and NOTCH targets.** Indicated mRNAs were analyzed by qRT-PCR. cDNAs were synthesized from RNA pooled from 3 mice. Values for each gene were normalized to Gapdh, and expressed relative to their own 0 h controls. *P < 0.05, **P < 0.01 (one-way ANOVA with TUKEY post-hoc test performed separately for each time point).

### DETAILED DESCRIPTION OF ILLUSTRATIVE EMBODIMENTS

The following description of the disclosure is merely intended to illustrate various embodiments of the disclosure. As such, the specific modifications discussed are not to be construed as limitations on the scope of the disclosure. It will be apparent to one skilled in the art that various equivalents, changes, and modifications may be made without departing from the scope of the disclosure, and it is understood that such equivalent embodiments are to be included herein.

### I. Definition

It is to be understood that both the foregoing general description and the following detailed description are exemplary and explanatory only and are not restrictive of the invention as claimed. In this application, the use of the singular includes the plural unless specifically stated otherwise. In this disclosure, the term "or" is used to mean "and/or" unless explicitly indicated to refer to alternatives only or the alternatives are mutually exclusive. As used herein "another" may mean at least a second or more. Furthermore, the use of the term "including", as well as other forms, such as "includes" and "included", is not limiting. Also, terms such as "element" or "component" encompass both elements and components comprising one unit and elements and components that comprise more than one subunit unless specifically stated otherwise. Also, the use of the term "portion" can include part of a moiety or the entire moiety.

As used herein, the singular forms "a", "an" and "the" include plural references unless the context clearly dictates otherwise.

As used herein, the term "apoptosis" refers to a form of programmed cell death that occurs in multicellular organisms. Apoptosis is a highly regulated and controlled process that can be initiated through an intrinsic pathway when the cell senses cell stress, or through an extrinsic pathway due to signals from other cells. During the process of apoptosis, a cell undergoes a series of characteristic changes including blebbing, cell shrinkage, nuclear fragmentation, chromatin condensation, chromosomal DNA fragmentation, and global mRNA decay.

As used herein, the term "cancer" refers to any diseases involving an abnormal cell growth and includes all stages and all forms of the disease that affects any tissue, organ or cell in the body. The term includes all known cancers and neoplastic conditions, whether characterized as malignant, benign, soft tissue, or solid, and cancers of all stages and grades including pre- and post-metastatic cancers. In general, cancers can be categorized according to the tissue or organ from which the cancer is located or originated and morphology of cancerous tissues and cells. As used herein, cancer types include, acute lymphoblastic leukemia (ALL), acute myeloid leukemia, adrenocortical carcinoma, anal cancer, astrocytoma, childhood cerebellar or cerebral, basal-cell carcinoma, bile duct cancer, bladder cancer, bone tumor, brain cancer, breast cancer, Burkitt's lymphoma, cerebellar astrocytoma, cerebral astrocytoma/malignant glioma, cervical cancer, chronic lymphocytic leukemia, chronic myelogenous leukemia, colon cancer, emphysema, endometrial cancer, ependymoma, esophageal cancer, Ewing family of tumors, Ewing's sarcoma, gastric (stomach) cancer, glioma, head and neck cancer, heart cancer, Hodgkin lymphoma, islet cell carcinoma (endocrine pancreas), Kaposi sarcoma, kidney cancer (renal cell cancer), laryngeal cancer, leukaemia, liver cancer, lung cancer, medulloblastoma, melanoma, neuroblastoma, non-Hodgkin lymphoma, ovarian cancer, pancreatic cancer, pharyngeal cancer, prostate cancer, rectal cancer, renal cell carcinoma (kidney cancer), retinoblastoma, , skin cancer, stomach cancer, supratentorial primitive neuroectodermal tumors, testicular cancer, throat cancer, thyroid cancer, vaginal cancer, visual pathway and hypothalamic glioma.

A "cell", as used herein, can be prokaryotic or eukaryotic. A prokaryotic cell includes, for example, bacteria. A eukaryotic cell includes, for example, a fungus, a plant cell, and an animal cell. The types of an animal cell *(e.g.,* a mammalian cell or a human cell) includes, for example, a cell from circulatory/immune system or organ, *e.g.,* a B cell, a T cell (cytotoxic T cell, natural killer T cell, regulatory T cell, T helper cell), a natural killer cell, a granulocyte (e.g., basophil granulocyte, an eosinophil granulocyte, a neutrophil granulocyte and a hypersegmented neutrophil), a monocyte or macrophage, a red blood cell (*e*.*g*., reticulocyte), a mast cell, a thrombocyte or megakaryocyte, and a dendritic cell; a cell from an endocrine system or organ, *e.g.,* a thyroid cell (*e.g.,* thyroid epithelial cell, parafollicular cell), a parathyroid cell (*e.g.,* parathyroid chief cell, oxyphil cell), an adrenal cell (*e.g.,* chromaffin cell), and a pineal cell (*e.g.,* pinealocyte); a cell from a nervous system or organ, *e.g.,* a glioblast (*e.g.,* astrocyte and oligodendrocyte), a microglia, a magnocellular neurosecretory cell, a stellate cell, a boettcher cell, and a pituitary cell (*e.g*., gonadotrope, corticotrope, thyrotrope, somatotrope, and lactotroph); a cell from a respiratory system or organ, *e.g.,* a pneumocyte (a type I pneumocyte and a type II pneumocyte), a clara cell, a goblet cell, and an alveolar macrophage; a cell from circular system or organ (*e.g.,* myocardiocyte and pericyte); a cell from digestive system or organ, *e.g.,* a gastric chief cell, a parietal cell, a goblet cell, a paneth cell, a G cell, a D cell, an ECL cell, an I cell, a K cell, an S cell, an enteroendocrine cell, an enterochromaffin cell, an APUD cell, and a liver cell (*e.g.,* a hepatocyte and Kupffer cell); a cell from integumentary system or organ, *e.g.,* a bone cell (*e.g.,* an osteoblast, an osteocyte, and an osteoclast), a teeth cell (*e.g.,* a cementoblast, and an ameloblast), a cartilage cell (*e.g.,* a chondroblast and a chondrocyte), a skin/hair cell (*e.g*., a trichocyte, a keratinocyte, and a melanocyte (Nevus cell), a muscle cell (*e*.*g*., myocyte), an adipocyte, a fibroblast, and a tendon cell; a cell from urinary system or organ (*e.g.,* a podocyte, a juxtaglomerular cell, an intraglomerular mesangial cell, an extraglomerular mesangial cell, a kidney proximal tubule brush border cell, and a macula densa cell); and a cell from reproductive system or organ (*e.g.,* a spermatozoon, a Sertoli cell, a leydig cell, an ovum, an oocyte). A cell can be normal, healthy cell; or a diseased or unhealthy cell (*e.g.,* a cancer cell). A cell further includes a mammalian zygote or a stem cell which include an embryonic stem cell, a fetal stem cell, an induced pluripotent stem cell, and an adult stem cell. A stem cell is a cell that is capable of undergoing cycles of cell division while maintaining an undifferentiated state and differentiating into specialized cell types. A stem cell can be an omnipotent stem cell, a pluripotent stem cell, a multipotent stem cell, an oligopotent stem cell and a unipotent stem cell, any of which may be induced from a somatic cell. A stem cell may also include a cancer stem cell. A mammalian cell can be a rodent cell, *e.g.,* a mouse, rat, hamster cell. A mammalian cell can be a lagomorpha cell, *e.g.,* a rabbit cell. A mammalian cell can also be a primate cell, *e.g*., a human cell.

As used herein, the term "chemotherapy" refers to a cancer treatment that uses one or more anti-cancer drugs. The anti-cancer drugs include, without limitation, Avastin, Bevacizumab, Camptosar (Irinotecan hydrochloride), Capecitabine, Cetuximab, Cyramza, Eloxatin (Oxaliplatin), Erbitux, 5-FU (fluorouracil), Fusilev (Leucovorin calcium), Ipilimumab, Keytruda, Lonsurf (Trifluridine and Tipiracil hydrochloride), Nivolumab (Opdivo), Panitumumab, Pembrolizumab, Ramucitrumab, Regorafenib, Stivarga, Ziv-Aflibercept.

As used herein, the term "organoid" refers to an *in vitro* three-dimensional culture that resembles a miniaturized and simplified organ and shows realistic micro-anatomy. Organoids are derived from one or a few cells from a tissue, embryonic stem cells or induced pluripotent stem cells through self-renewal and differentiation.

As used herein, the term "radiotherapy" refers to a therapy using ionizing radiation to control or kill malignant cells. Radiotherapy is normally delivered by a linear accelerator. Radiotherapy may be used to treat a number of types of cancer if they are located to one area of the body. Radiotherapy may also be use as part of adjuvant therapy, to prevent tumor recurrence after surgery to remove a primary malignant tumor. Radiotherapy may be synergistic with chemotherapy, and has been used before, during, and after chemotherapy in susceptible cancers.

As used herein, the term "subject" refers to a human or any non-human animal (*e*.*g*., mouse, rat, rabbit, dog, cat, cattle, swine, sheep, horse or primate). A human includes pre- and post-natal forms. In many embodiments, a subject is a human being. A subject can be a patient, which refers to a human presenting to a medical provider for diagnosis or treatment of a disease. The term "subject" is used herein interchangeably with "individual" or "patient." A subject can be afflicted with or is susceptible to a disease or disorder but may or may not display symptoms of the disease or disorder.

The term "therapeutically effective amount" or "effective dosage" as used herein refers to the dosage or concentration of a drug effective to treat a disease or condition. For example, with regard to the use of the small-molecule compounds thereof disclosed herein to treat GI injury induced by a chemotherapy or radiotherapy, a therapeutically effective amount is the dosage or concentration of the compound capable of preventing or ameliorating the GI injury induced ty the chemotherapy or radiotherapy.

"Treating" or "treatment" of a condition as used herein includes preventing or alleviating a condition, slowing the onset or rate of development of a condition, reducing the risk of developing a condition, preventing or delaying the development of symptoms associated with a condition, reducing or ending symptoms associated with a condition, generating a complete or partial regression of a condition, curing a condition, or some combination thereof.

### II. Treatment-induced Gastrointestinal Injury

The gastrointestinal (GI) epithelium is the fastest renewing adult tissue and is maintained by tissue-specific stem cells. Treatment-induced GI side effects are a major dose-limiting factor for chemotherapy and abdominal radiotherapy and can decrease the quality of life in cancer patients and survivors. For example, 5-fluorouracil (5-FU), which is commonly used to treat colorectal cancer patients, has been reported to cause chemotherapy induced diarrhea in 50% of patients. While radiation and most chemotherapeutic agents damage multiple tissues and organ systems, irinotecan hydrochloride (CPT-11), which is commonly used in combination with 5-FU to treat colorectal cancer patients, causes selective GI injury, with severe diarrhea and nausea in more than 50% of patients. This acute toxicity is caused by the accumulation of high concentration of SN-38, the active metabolite of CPT-11, in the intestine, as well as the conversion of nontoxic SN-38 glucuronide back to SN-38 by gut bacteria, followed by intestinal reabsorption. SN-38 is an inhibitor of topoisomerase I, a key enzyme in both DNA replication and RNA transcription, and causes replication stress and DNA damage in proliferating cells. Anti-diarrhea medication can help alleviate CPT-11-induced diarrhea but has a limited effect on reducing long-term GI dysfunction. There is currently no US FDA-approved agent that prevents or treats CPT-11-induced GI injury or complication.

Chemotherapy or radiation-induced acute enteropathy is characterized by loss of proliferating crypt cells, epithelial barrier breakdown, and inflammation during or shortly after the treatment. In many patients, delayed enteropathy can occur months or later after therapy and is characterized by intestinal dysfunction associated with pathological changes in the epithelial and stromal compartments, such as vascular sclerosis and progressive intestinal wall fibrosis. Emerging evidence suggests that chronic intestinal damage is likely a consequence of early toxicity. Enterotoxicity caused by chemotherapeutics, such as CPT-11 and 5-FU, is associated with rapid crypt apoptosis in mice and humans. The small intestinal epithelium is the fastest renewing adult tissue, and intestinal stem cells (ISCs) located in the bottom of crypts drive the renewal and regeneration after injury. ISCs include the columnar cells at the crypt bottom (CBCs) and some cells at position 4 relative to the crypt bottom (+4 cells). Genetic ablation of leucine-rich repeat-containing G protein (heterotrimeric guanine nucleotide-binding protein)-coupled receptor 5-expressing (LGR5+) CBCs is well tolerated in healthy mice but strongly exacerbates radiation-induced intestinal injury, although underlying mechanisms remain unknown.

Activation of p53 after DNA damage results in tissue- and target-specific outcomes such as tumor suppression or acute toxicity. p53 activation is a double-edged sword in controlling intestinal regeneration after high-dose radiation. On one hand, p53-dependent PUMA induction accounts for most radiation-induced apoptosis and acute loss of intestinal and hematopoietic stem and progenitor cells, and PUMA deficiency or down-regulation protects against radiation-induced lethality and lymphomagenesis. However, p53-dependent induction of p21, and perhaps other targets, is essential for productive intestinal regeneration by preventing DNA damage accumulation, replication stress, and delayed nonapoptotic cell death, and p53/p21 deficiency exacerbates intestinal injury. The role of p53 in chemotherapy-induced GI injury is not well understood. The p53 inhibitor pifithrin-α reduced CPT-11-induced intestinal apoptosis within the first several hours in rats but did not affect delayed cell death or the onset of mucositis.

### III. Therapeutic Compounds

As disclosed herein, the inventors surprisingly discovered that pharmacological inhibition of Puma by a group of small-molecule compounds, but not p53 deficiency, provides potent protection against GI injury induced by single and repeated exposures to CPT-11 but does not compromise its antitumor activity in vivo. This protection is associated with selective preservation of LGR5⁺ stem cells, stem cell niche, and genome integrity.

In certain embodiments, the small-molecule compound is selected from the group consisting of:

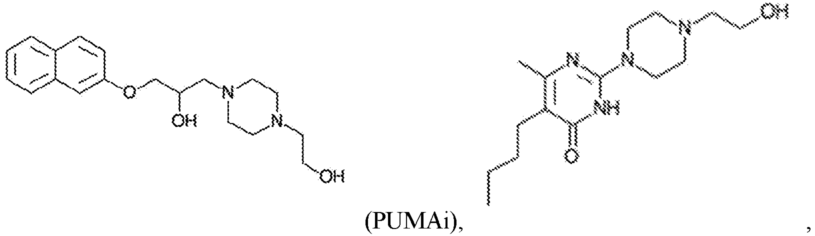

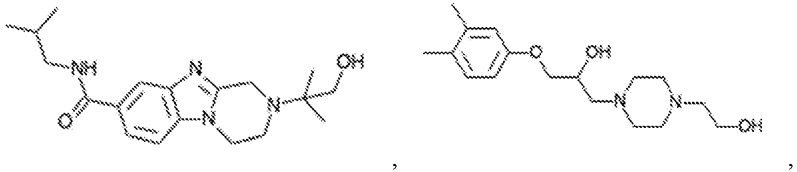

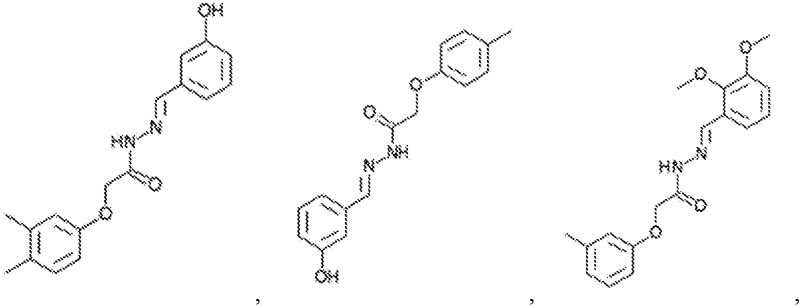

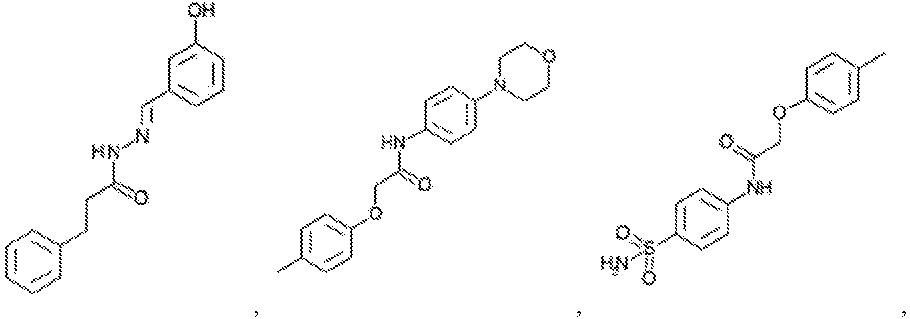

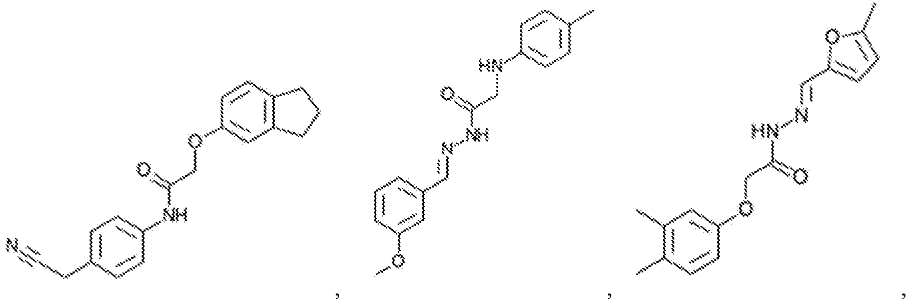

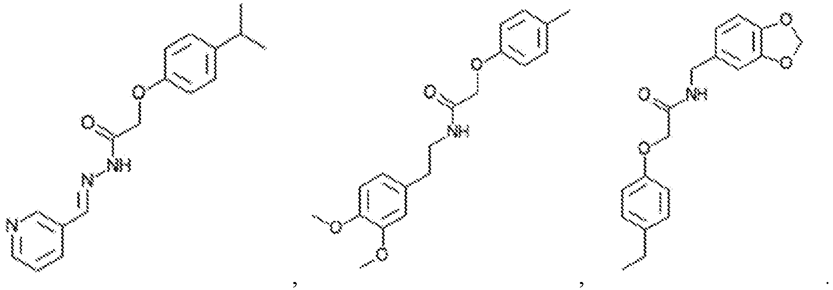

In certain embodiments, the compounds described above are deuterated. As used herein, a "deuterated compound" refers to a compound in which one or more of the hydrogen atoms have been replaced by deuterium isotope. It is understood that some variation of natural isotopic abundance occurs in a synthesized compound depending on the origin of chemical materials used in the synthesis. Thus, a preparation of the compound will inherently contain small amount of deuterated compounds. The concentration of naturally abundant stable hydrogen and carbon isotopes is small and immaterial as compared to the degree of stable isotopic substitution of the deuterated compounds described herein. In a deuterated compound described herein, when a particular position is designated as having deuterium, it is understood that the abundance of deuterium at the position is substantially greater than the natural abundance of deuterium, which is 0.015%. A position designated as having deuterium typically has a minimum isotopic enrichment factor of at least 3000 (45% deuterium incorporation), e.g., at least 3500 (52.5% deuterium incorporation), at least 4000 (60% deuterium incorporation), at least 4500 (67.5% deuterium incorporation), at least 5000 (75% deuterium), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

In certain embodiments, the deuterated compound disclosed herein has a structure of 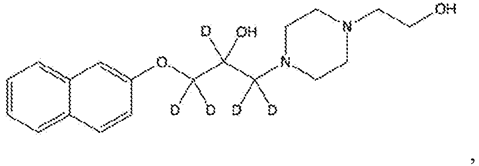 wherein "D" refers to deuterium.

The synthesis of the small-molecule compounds described herein can be readily achieved by synthetic chemists of ordinary skill using methods known in the art. Such methods can be carried out utilizing corresponding deuterated and optionally, other isotope-containing reagents and/or intermediates to synthesize the compounds delineated herein, or invoking standard synthetic protocols known in the art for introducing isotopic atoms to a chemical structure.

### IV. Formulation and Administration

The present disclosure provides pharmaceutical compositions used to prevent or treat treatment-induced GI injury. Such compositions comprise a prophylactically or therapeutically effective amount of a compound disclosed herein, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. Such pharmaceutical carriers can be sterile liquids, such as water and oils, including those of petroleum, animal, vegetable or synthetic origin, such as peanut oil, soybean oil, mineral oil, sesame oil and the like. Water is a particular carrier when the pharmaceutical composition is administered intravenously. Saline solutions and aqueous dextrose and glycerol solutions can also be employed as liquid carriers, particularly for injectable solutions. Other suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like.

The composition, if desired, can also contain minor amounts of wetting or emulsifying agents, or pH buffering agents. These compositions can take the form of solutions, suspensions, emulsion, tablets, pills, capsules, powders, sustained-release formulations and the like. Oral formulations can include standard carriers such as pharmaceutical grades of mannitol, lactose, starch, magnesium stearate, sodium saccharine, cellulose, magnesium carbonate, *etc.* Examples of suitable pharmaceutical agents are described in "Remington's Pharmaceutical Sciences." Such compositions will contain a prophylactically or therapeutically effective amount of the compound together with a suitable amount of carrier so as to provide the form for proper administration to the patient. The formulation should suit the mode of administration, which can be oral, intravenous, intraarterial, intrabuccal, intranasal, nebulized, bronchial inhalation, or delivered by mechanical ventilation.

The compound of the present disclosure, as described herein, can be formulated for parenteral administration, *e.g.,* formulated for injection *via* the intradermal, intravenous, intramuscular, subcutaneous, intra-tumoral or even intraperitoneal routes. The compound could alternatively be administered by a topical route directly to the mucosa, for example by nasal drops, inhalation, or by nebulizer. Pharmaceutically acceptable salts include the acid salts and those which are formed with inorganic acids such as, for example, hydrochloric or phosphoric acids, or such organic acids as acetic, oxalic, tartaric, mandelic, and the like. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

Generally, the ingredients of compositions of the present disclosure are supplied either separately or mixed together in unit dosage form, for example, as a dry lyophilized powder or water-free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compositions of the present disclosure can be formulated as neutral or salt forms. Pharmaceutically acceptable salts include those formed with anions such as those derived from hydrochloric, phosphoric, acetic, oxalic, tartaric acids, *etc.,* and those formed with cations such as those derived from sodium, potassium, ammonium, calcium, ferric hydroxides, isopropylamine, triethylamine, 2-ethylamino ethanol, histidine, procaine, *etc.*

The compound or the pharmaceutical composition disclosed herein can be administered to a subject in a therapeutically effective amount so as to prevent or treat the treatment-induced injury. In certain embodiments, the therapeutic effective amount is about 0.001 to about 10 mg/kg body weight, wherein the composition is administered once a day, twice a day, three times a day, once a week, twice a week, three times a week, or once per two weeks. In certain embodiments, the effective amount is about 0.01 mg/kg body weight, 0.1mg/kg body weight, about 0.5mg/kg body weight, about 1mg/kg body weight, about 1.5mg/kg body weight, about 2mg/kg body weight, about 2.5mg/kg body weight, about 3mg/kg body weight, about 3.5mg/kg body weight, about 4mg/kg body weight, about 4.5mg/kg body weight, about 5mg/kg body weight, about 5.5mg/kg body weight, or about 6mg/kg body weight. In certain embodiments, the unit dose of the composition is between about 0.001 mg to about 10mg (e.g. about 0.005 mg, about 0.01 mg, about 0.05mg, about 0.1 mg, about 0.5mg, about 1 mg, about 2 mg, about 3 mg, about 4 mg, about 5 mg, about 6 mg, about 7 mg, about 8 mg, about 9 mg, about 10 mg). In certain embodiments, the effective amount is 0.01 mg/kg body weight, 0.1 mg/kg body weight, 0.5mg/kg body weight, or 1mg/kg body weight, wherein the composition is administered once a day. In certain embodiments, the effective amount is 5mg/kg body weight, wherein the composition is administered once a week.

### V. Examples

The following examples are included to demonstrate illustrative embodiments of the invention. It should be appreciated by those of skill in the art that the techniques disclosed in the examples which follow represent techniques discovered by the inventor to function well in the practice of the invention, and should only be considered to constitute illustrative modes for its practice.

### Example 1. Materials and Methods

### Compound screening and preparation

Candidate PUMA inhibitor structures and analogs were identified using *in silico* screening of the ZINC 8.0 database, and *in silico* ADME/Toxicity profiles as described previously (Mustata G. et al. Curr Top Med Chem (2011) 11:281-290). Compounds related to PUMAi were obtained from commercial vendors.

### Study design

The goals of this study were to determine whether PUMA- and p53-dependent apoptosis mediates chemotherapy-induced intestinal stem cell loss and enteropathy and whether its genetic and pharmacological inhibition provides intestinal chemoprotection. Non-tumor-bearing and tumor-bearing mice, p53 and Puma KO mice, a small-molecule PUMAi, and in vitro mouse and human colonic organoids were used to measure the effects of PUMA inhibition on intestinal injury and regeneration, as well as tumor responses after chemotherapy. Sample size was determined using published work and power calculations. Experimenters were not blinded to treatment groups during the acquisition of data.

### Mice and treatment

The procedures for all animal experiments were approved by the Institutional Animal Care and Use Committee of the University of Pittsburgh. Puma+/+ (WT) and Puma-/- (Puma KO) mice were generated in-house by heterozygote breeding. WT and Puma KO alleles were genotyped from genomic DNA isolated from tail snips, as described . p53-/- (p53 KO) mice were purchased from the Jackson Laboratory. The LGR5 marking mice Lgr5-EGFP (Lgr5-EGFP-IRES-creERT2) have been described. All strains are in or have been backcrossed with the C57BL/6 background for more than 10 generations (F10). Mice were housed in microisolator cages in a room illuminated from 07:00 to 19:00 hours (12:12-hour light/dark cycle), with access to water and chow ad libitum. CPT-11 (irinotecan hydrochloride; Camptosar, Pfizer) treatments were administered at 200 mg/kg in saline, unless otherwise indicated, by intraperitoneal injection (200 ul for a 20-g mouse). PUMAi was custom-synthesized by ChemBridge with a minimum purity of 95% by HPLC/mass spectrometry (MS), prepared as a stock (50 mg/ml) in dimethyl sulfoxide, then freshly diluted in phosphate-buffered saline (PBS) (2 mg/ml), and given at 10 mg/kg intraperitoneally (200 ul for a 20-g mouse).

For tumor experiments, 4 million LLC cells [American Type Culture Collection (ATCC)] were injected into the flanks of WT and Puma KO mice and allowed to grow for 11 days. Mice were then treated with CPT-11 (200 mg/kg) three times per week for 2 weeks. PUMAi (10 mg/kg) or vehicle control was administered by intraperitoneal injection 2 hours before and 20 hours after each dose of CPT-11. Mice were sacrificed 4 hours after the final CPT-11 treatment.

CPT-11 (irinotecan hydrochloride; Camptosar, Pfizer) treatments were administered at 200 mg/kg i.p. unless otherwise indicated. PUMA inhibitor (PUMAi) was prepared as a 50 mg/ml stock in DMSO, then freshly diluted to 2 mg/ml in PBS, and given at 10 mg/kg i.p. 2 hours before chemotherapy treatment unless otherwise indicated. For survival experiments, mice were treated with CPT-11 for three consecutive days. PUMAi was given 4 times, 2 hours before each chemotherapy and 22 hours after the last.

For tumor experiments, mice were treated with CPT-11 three times per week for two weeks. Mice receiving PUMAi were treated 2 hours before each dose of CPT-11 and 20 hours after. Mice were sacrificed four hours after the final CPT-11 treatment **(****FIG. 13A****).** Tumor volumes were measured with calipers and calculated as volume = (length x width2)/2. Mice were 7-9 weeks old, and roughly equal numbers of male and female animals were used. To account for variations in starting body weights, the values in Figures 3 and 4 are expressed as a percentage of the respective mouse weights at the time of the first CPT-11 treatment.

5-fluorouracil (5-FU) (APP Pharmaceuticals) treatments were administered at 200 mg/kg i.p. For total body irradiation experiments, PUMAi (10 mg/kg) was given 30 minutes before and 30 minutes after irradiation, followed by daily doses for four days.

### Tissue processing and histological analysis

Immediately after sacrifice, about 10-cm portion of the jejunum was removed and carefully rinsed with ice-cold saline. The tissue was opened longitudinally and tacked to a foam board for fixation overnight in 10% formalin. Tissues were then rolled up into "swiss rolls" and embedded in paraffin. For tumor analysis, flank tumors were removed, measured, and cut into pieces for either formalin fixation or flash freezing in a dry ice/ethanol bath. Tissue sections (5 um) were deparaffinized and rehydrated through graded ethanols. Histological analysis was performed by hematoxylin and eosin (H&E) staining.

Average villus height was determined by measuring 40 to 50 villi from different locations of the small intestine, from at least three different animals per group, and reported as means Å} SEM. Measurements were made from the top of the crypt to the tip of the villus using 100Å~ H&E images and SPOT 5.1 Advanced software (Diagnostic Instruments Inc.).

### Immunohistochemistry and immunofluorescence

Rehydrated sections were treated with 3% hydrogen peroxide (immunochemistry only), followed by antigen retrieval for 10 min in boiling 0.1 M citrate buffer (pH 6.0) with 1 mM EDTA. Apoptosis was analyzed by TUNEL staining with the ApopTag Peroxidase In Situ Apoptosis Detection kit (Chemicon International) according to the manufacturer's instructions.

*Active caspase 3 IHC.* Sections were deparaffinized and rehydrated through graded ethanols. Rehydrated sections were then treated with 3% hydrogen peroxide, followed by antigen retrieval for 10 minutes in boiling 0.1 M citrate buffer (pH 6.0) with 1 mM EDTA. Non-specific antibody binding was blocked using 20% goat serum (Invitrogen) at room temperature for 30 minutes. Sections were incubated overnight at 4oC in a humidified chamber with 1:100 diluted rabbit-anticaspase-3 (cleaved, Asp 175) (9661; Cell Signaling Technologies). Sections were then incubated for 1 hour at room temperature with biotinylated goat anti-rabbit secondary antibodies (#31822; Pierce) and developed with an ABC kit and DAB (Vector Laboratories).

*LGR5 (GFP) IF.* Sections were deparaffinized and rehydrated through graded ethanols. Antigen retrieval was performed by boiling for 10 minutes in 0.1 M citrate buffer (pH 6.0) with 1 mM EDTA. Non-specific antibody binding was blocked using 20% goat serum (Invitrogen) at room temperature for 30 min. Sections were incubated overnight at 4oC in a humidified chamber with 1:50 diluted mouse anti-GFP (sc-9996; Santa Cruz Biotechnology). Sections were then incubated with AlexaFluor 488-conjugated goat anti-mouse secondary antibodies (1:200; AA11001; Invitrogen) for 1 hour at room temperature. Sections were then washed in PBS and mounted with VectaShield + DAPI (Vector Labs).

*LGR5(GFP)*/*TUNEL IF.* Sections were prepared as described above. Non-specific antibody binding was blocked using 20% goat serum (Invitrogen) at room temperature for 30 min. Sections were incubated overnight at 4oC in a humidified chamber with 1:50 diluted mouse anti-GFP (sc-9996; Santa Cruz Biotechnology). Sections were then incubated with AlexaFluor 594-conjugated goat anti-mouse secondary antibodies (1:200; AA11005; Invitrogen) for 1 hour at room temperature. Sections were then washed in PBS, and TUNEL staining was performed with the ApopTag Fluorescein In Situ Apoptosis Detection Kit (Chemicon) according to the manufacturer's instructions.

*MMP7 IF.* Sections were prepared as described above. Non-specific antibody binding was blocked using 20% chicken serum (Invitrogen) at room temperature for 30 min. Sections were washed in PBS and incubated overnight at 4oC in a humidified chamber with 1:100 diluted goat anti-MMP7 (AF2967; R&D Systems). Sections were then incubated with AlexaFluor 594-conjugated rabbit anti-goat secondary antibodies (1:200; AA11080; Invitrogen) for 1 hour at room temperature. Sections were then washed in PBS and mounted with VectaShield + DAPI (Vector Labs) for visualization.

*LGR5(GFP)*/*MMP7 IF.* Sections were prepared as described above. Non-specific antibody binding was blocked using 20% chicken serum (Invitrogen) at room temperature for 30 min. Sections were then stained for GFP as described above, followed by staining for MMP7 as described.

*PCNA IF.* Sections were prepared as described above. Non-specific antibody binding was blocked using 20% goat serum (Invitrogen) at room temperature for 30 min. Sections were incubated overnight at 4oC in a humidified chamber with 1:100 diluted mouse-anti-PCNA (sc-56; Santa Cruz). Sections were then incubated with AlexaFluor 594-conjugated goat anti-mouse secondary antibodies (1:200; Invitrogen) for 1 hour at room temperature. Sections were then washed in PBS and mounted with VectaShield + DAPI (Vector Labs).

*CD166 IF.* Sections were prepared as described above. Non-specific antibody binding was blocked using 20% rabbit serum (Pierce) at room temperature for 30 min. Sections were incubated overnight at 4oC in a humidified chamber with goat anti-mouse CD166 antibodies (1:100; AF1172; R&D Systems). Sections were then incubated with AlexaFluor-594 rabbit antigoat secondary antibodies (1:200; A11080; Invitrogen) for 1 hour at room temperature and counterstained with VectaShield plus DAPI.

*p53 BPI IF:* Sections were prepared as described above. Non-specific antibody binding was blocked using 20% goat serum (Invitrogen) at room temperature for 30 min. Sections were incubated overnight at 4oC in a humidified chamber with rabbit anti-mouse 53BP1 antibodies (1:100; IHC-00001; Bethyl Laboratories). Sections were then incubated with AlexaFluor-594 goat anti-rabbit secondary antibodies (1:200; A11012; Invitrogen) for 1 hour at room temperature and counterstained with VectaShield plus DAPI.

*Neutrophil IF.* Sections were prepared as described above. Non-specific antibody binding was blocked using 20% goat serum (Invitrogen) at room temperature for 30 min. Sections were incubated overnight at 4oC in a humidified chamber with rat anti-mouse Ly-6B.2 (1:100; MCA771GT; AbD Serotec). Sections were then incubated with AlexaFluor-594 goat antirat secondary antibodies (1:200; A11007; Invitrogen) for 1 hour at room temperature and counterstained with VectaShield plus DAPI.

### LC-MSIMS quantitation of PUMAi

Acetonitrile and water (all HPLC grade) were purchased from Fisher Scientific. Formic acid and trifluoroacetic acid was purchased from Sigma-Aldrich. Stock solutions of PUMAi and [D5]-PUMAi internal standard were prepared at 1 mg/mL in DMSO, diluted 10 times in acetonitrile, and then stored at -80 °C. On the day of the assay, the solution was serially diluted (in 10-fold steps) in acetonitrile to obtain the lower calibration working solutions of 0.01 and 0.001 mg/mL. These calibration working solutions were diluted in mouse plasma (Lampire Biological Laboratories, Inc.) to produce the following analyte concentrations: 10, 30, 50, 100, 300, 500, 1000, 3000 ng/mL. For each calibration series, zero (containing 10 ng/mL of internal standard) and blank samples were also prepared from 50 µL of Lampire plasma. Quality control (QC) stock solutions were diluted in Lampire mouse plasma to produce the following QC samples: QC Low (QCL) 20 ng/mL; QC Mid (QCM) 200 ng/mL, and QC High (QCH) 2500 ng/mL.

To extract PUMAi, we added 10 µL of internal standard solution (1000 ng/mL [DS]-PUMAi in acetonitrile) to 50 µL of plasma sample in a micro centrifuge tube. 250 µL of acetonitrile was added, and the samples were vortexed for 30 s. The sample was centrifuged at 12,000 x g for five min, and the resultant supernatant was evaporated to dryness under nitrogen at 34 °C in 12 x 75 mm glass tubes. The dry residue was reconstituted with 100 µL of acetonitrile/water (v/v), and 10 µL was injected into the LC-MS/MS system.

The LC-MS/MS system consisted of an Agilent 1100 autosampler and binary pump, and a Waters Quattromicro tandem mass spectrometer. The liquid chromatography was performed with a gradient mobile phase consisting of A: acetonitrile/0.1% formic acid (v/v) and B: water/0.1% formic acid (v/v). The mobile phase was pumped at a flow rate of 0.3 mL/min, and separation was achieved using a Phenomenex Luna 3µm PFP (100a 150x2 mm) column. The gradient mobile phase was as follows: from 0 to 5 min, solvent A was 15%. Solvent A was then increased linearly to 80% at 12 min and maintained at 80% until 16 min. At 16.1 min, solvent A was decreased to 15% and maintained at 15% until 22 min. The run time was 22 min.

The mass spectrometer was operated in ESI positive mode using MRM detection. The parameters of the mass spectrometer were as follows; capillary 1.0 kV, cone voltage 40V, desolvation temperature 400 °C, desolvation gas flow 550 L/h, cone gas flow 50 L/h, LM and HM resolution 12, collision energy 25 V, and gas cell pirani pressure 1.5*10⁻³ mbar. The mass transitions monitored were m/z 331>143 for PUMAi and 345>157 for the internal standard.

To assess recovery from plasma, we compared areas from control plasma spiked with 1000 ng/mL of PUMAi, prepared as described above, with the areas obtained with a neat solution of PUMAi in water. Intestinal mucosa was analyzed after dilution in control plasma to within the calibration range. Intestinal mucosa was collected by scraping from a 10 cm portion of the jejunum, and stored at -80C until use. Three mice were used for each treatment group.

### In situ hybridization

ISH for Olfm4 was carried out with the RNAscope 2.0 BROWN kit (Advanced Cell Diagnostics) according to the manufacturer's instructions. Briefly, tissue sections were baked for 60 min at 60ÅãC, then deparaffinized in xylene, and rehydrated through graded ethanols. Sections then underwent three pretreatment steps, probe hybridization, six amplification steps, development, and counterstaining. ISH for Puma was carried out as previously described.

### Western blotting

Fresh mucosal scrapings or minced tumor tissues were washed in 1 ml of ice-cold PBS and pelleted at 400g. Pellets were resuspended in 700 ul of homogenization buffer (0.25 M sucrose, 10 mM Hepes, and 1 mM EGTA) supplemented with protease inhibitors (cOmplete EDTAfree mini, Roche) and homogenized in a Dounce homogenizer with 50 strokes of the pestle. After clearing by centrifugation at 16,000g, protein concentrations in the supernatant were determined by a spectrophotometer (NanoDrop 2000, Thermo Fisher Scientific).

Proteins (30 ug) were separated by SDS-polyacrylamide gel electrophoresis using the NuPAGE system (Invitrogen) and transferred to polyvinylidene difluoride membranes (Immobilon-P, Millipore). Representative results are shown, and similar results were obtained in at least three independent experiments. Antibodies used include PUMA (ab9643, Abcam), p53, p21, hemagglutinin (HA) (sc-6243, sc-397, and sc-805; Santa Cruz Biotechnology), V5 (R960-25, Invitrogen), tubulin (CP06, Oncogene Science), and actin (A5541, Sigma-Aldrich).

### Quantitative real-time polymerase chain reaction

Fresh mucosal scrapings from 10 cm of jejunum were washed in cold PBS, resuspended in 700 ul of RNA lysis buffer, and homogenized in a Dounce homogenizer. RNA was isolated using the Quick-RNA MiniPrep kit (Zymo Research) according to the manufacturer's instructions. Organoids were freed from Matrigel using Cell Recovery Solution (Corning), as described in, before RNA isolation. Complementary DNA was generated from 2 ug of total RNA from mice or ~100 ng of total RNA from colonic organoids and was pooled from three mice or three wells of organoid culture per treatment group using SuperScript III reverse transcriptase (Invitrogen) and random primers. Gene expression was normalized to Gapdh. Representative results are shown, and similar results were obtained in at least three independent experiments. Details on mouse and human primers are found in Tables 1 and 2.

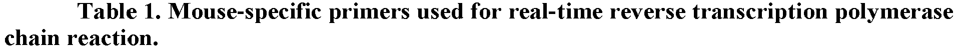

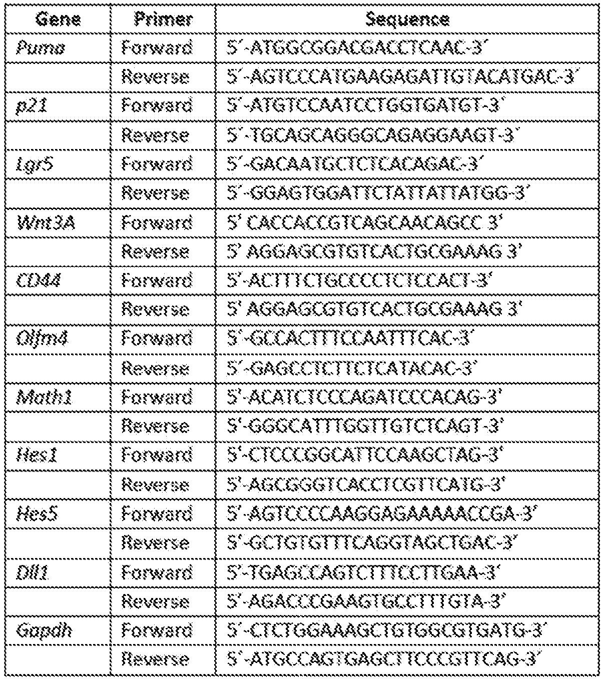

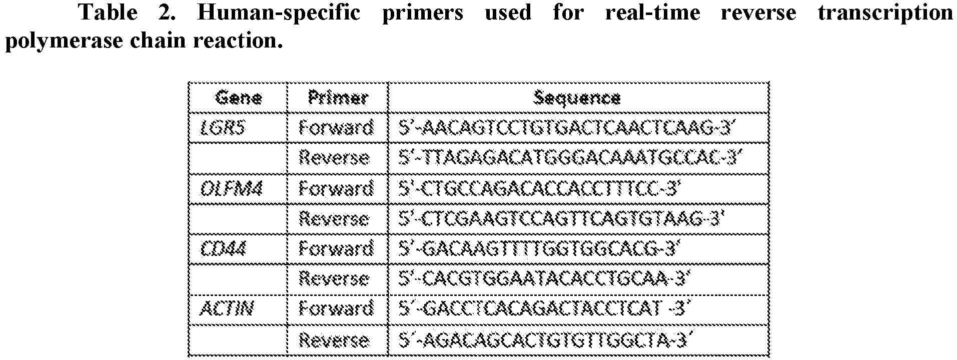

### Cells and treatment

HCT 116, DLD1, SW480, and HT29 human colon cancer cells (ATCC), as well as HCT 116 p53 KO and PUMA KO, were maintained at 37 °C and 5% CO2 in McCoy's 5A medium (Invitrogen) supplemented with 10% defined fetal bovine serum (HyClone), penicillin (100 U/ml), and streptomycin (100 ug/ml) (Invitrogen). Human embryonic kidney 293 cells (ATCC) were maintained in Dulbecco's modified Eagle's medium (Invitrogen) with the same supplements. For treatments, cells were plated in 12-well plates at ~30% density for 24 hours and then treated with CPT (Sigma-Aldrich). PUMAi was added to cells at 25 uM at the same time as CPT.

### Measurement of apoptosis

After treatment, floating and adhering cells were collected and stained in a solution containing 3.7% formaldehyde, 0.5% NP-40, and Hoechst 33258 (10 ug/ml) (Molecular Probes) in PBS. Apoptosis was assessed through microscopic visualization of condensed and fragmented nuclei, as previously described (Yu J. et al., Proc Natl Acad Sci U S A (2003) 100:1931-36). A minimum of 300 cells per treatment were analyzed in triplicate. Representative results are shown, and similar results were obtained in at least three independent experiments.

### Immunoprecipitation

HEK293 cells were transfected with previously described plasmids expressing HA-tagged PUMA (HA-PUMA), HA-tagged inactive PUMA (ΔBH3), or V5-tagged Bcl-xL (V5-Bcl-xL) using Lipofectamine 2000 according to the manufacturer's instructions. To test the PUMAi, 293 lysates containing HA-PUMA or HA-ΔBH3 were incubated for 15 min with 25 uM PUMAi, then mixed with V5-Bcl-xL lysates for 1 hour, and subjected to immunoprecipitation with an anti-HA antibody (sc-805, Santa Cruz Biotechnology). BIM/Mcl-1 interaction was tested in a similar fashion using lysates containing HA-BIM and V5-Mcl-1. Representative results are shown, and similar results were obtained in at least three independent experiments.

### Measurement of PUMAi in tissues

PUMAi {1-[4-(2-hydroxyethyl)-1-piperazinyl]-3-(2-naphthyloxy)-2-propanol dihydrochloride} was synthesized by ChemBridge, with a purity of 95% by HPLC-MS, and an internal isotopic standard PUMAih7 (D5, five internal hydrogens replaced by deuterium) was synthesized by Alsachim, with purity approaching 100% by HPLC-MS. Nuclear magnetic resonance confirmed the isotope enrichment by 99.4% or more. Pooled plasma from untreated mice was used as control plasma. Detailed methods on LC-MS/MS (liquid chromatography-tandem MS) quantitation of PUMAi are found in Supplementary Materials and Methods.

### Mouse and human colonic organoids

### Preparation of WRN-conditioned medium

L-WRN cells (ATCC^{®} CRL-3276) were cultured in DMEM (Invitrogen) supplemented with 1Xpenicillin/streptomycin (Invitrogen), 0.5 mg/mL G418 (ant-gn-1, InvivoGen), 0.5 mg/mL hygromycin B (10687010, Invitrogen), and 10% FBS (vol/vol) until confluent. Cells were then collected with Trypsin-EDTA (Invitrogen) and split into three T75 cell culture flasks (Sarstedt) in DMEM supplemented with 1X penicillin/streptomycin and 10% FBS (HyClone) (vol/vol) until they became over-confluent. Cells were then rinsed with 5 ml of primary culture medium (Advanced DMEM/F12 (Invitrogen) supplemented with 1X penicillin/streptomycin, 2 mM GlutaMAX (ThermoFisher), 20% FBS [vol/vol]) and cultured in the same medium (15 ml/flask). Every 24 hours, the primary culture medium (conditioned medium) was collected, and fresh medium was added to the flasks. The conditioned medium was centrifuged at 2,000 g for 5 min at room temperature, and the supernatant was decanted and stored in aliquots at -20 °C until use.

### Mouse crypt isolation, organoids, and treatment

Mouse crypt isolation, organoid development, and passage were done as previously described with minor modifications. In brief, the complete culture medium for mouse intestinal organoids was slightly modified as follows: advanced Dulbecco's modified Eagle medium (DMEM)/F12 (12634-010, Invitrogen) was supplemented with 100 units/ml penicillin/0.1 mg/ml streptomycin (Invitrogen), 2 mM GlutaMAX (ThermoFisher), 20% (vol/vol) FBS (S11150, ATLANTA Biologicals), and WRN-conditioned medium (50%, vol/vol). Freshly isolated crypts were cultured with the same medium plus 10 µM Y-27632 (Y0503 Sigma) and 100 µg/ml Primocin (ant-pm-1, InvivoGen). For CPT and PUMAi treatment, intestinal organoids were passaged and re-plated in 25 µL of Matrigel (356231, Corning) in 24-well plates 24 hours before treatment with CPT (0.5 µM, C9911, Sigma-Aldrich) and PUMAi (50 µM) for 24 hours. The CPT-containing medium was replaced with complete medium containing PUMAi (50 µM). Three groups (treatment control, CPT only, and CPT plus PUMAi) were set up. Organoids 100 µm or greater in diameter were enumerated on day 6 after CPT treatment. Similar results were obtained from at least three independent experiments with triplicate wells in each experiment.

### Human crypt isolation, organoid development, and treatment

Surgically resected intestinal tissues were obtained from Health Science Tissue Bank (HSTB) of UPMC Shadyside Hospital and UPMC Presbyterian Hospital. All samples were obtained with informed consent and approval by the University of Pittsburgh Ethics Committee. Human normal colon crypt isolation and organoid development were performed as previously described with modifications. The complete culture medium for normal human colon organoids contains advanced DMEM/F12 (12634-010, Invitrogen) supplemented with 1X penicillin/streptomycin (15140-122, Invitrogen ), 10 mM HEPES (15630-106, Invitrogen), 2 mM GlutaMAX (Invitrogen), 1XB27 (17504-044, Invitrogen), 1XN2 (17502-048, Invitrogen), 1 mM N-Acetylcysteine (A0737, Sigma), 10 nM [leu-15]-Gastrin (G9145, Sigma), 10 mM nicotinamide (N0636, Sigma), 10 µM SB202190 (S7067, Sigma), 50 ng/ml recombinant murine EGF (315-09, Peprotech), 0.5 µM A83-01 (2939, Tocris Bioscience), 10 nM PGE2 (22-961-0, Tocris Bioscience), and 50% WRN-conditioned medium (vol/vol). Freshly isolated crypts were cultured with the same medium plus 10 µM Y-27632 and 100 µg/ml Primocin. Organoids 100 µm or greater in diameter were enumerated on day 6 after CPT treatment. Similar results were obtained from at least three independent experiments with triplicate wells in each experiment.

### Statistical analysis

Statistical analyses were performed with GraphPad Prism IV software. Multiple comparisons of the responses were analyzed by one-way analysis of variance (ANOVA) and Tukey's post hoc test, whereas those between two groups were made by two-tailed, unpaired t test. Survival data were analyzed by log-rank test. Differences were considered significant if the probability of the difference occurring by chance was less than 5 in 100 (P < 0.05). Sample size was determined using a combination of published work and power calculations. For ANOVA, the power for a test of interaction was computed in a two-way factorial design applied by constructing mixed linear growth models to calculate the needed sample size. It was estimated that usually 5 to 10 per group will provide 80% power to detect a standardized interaction of 1.5 SDs.

### Example 2. PUMA mediates chemotherapy-induced crypt apoptosis and lethality

To investigate a potential role of p53-dependent apoptosis in chemotherapy-induced acute intestinal injury, the inventors treated wild-type (WT), p53 knockout (KO), and Puma KO mice with a single dose of irinotecan (200 mg/kg; CPT-11). CPT-11 induced robust crypt apoptosis in WT mice, as measured by terminal deoxynucleotidyl transferase-mediated deoxyuridine triphosphate nick end labeling (TUNEL) and active caspase-3 staining, which peaked at 6 hours and decreased gradually by 48 hours. Apoptosis was observed in both the stem cells at the crypt base (CBCs at positions 1 to 3 relative to the crypt bottom) and +4 to 9 cells (relative to the crypt bottom), but it was abrogated in p53 KO and Puma KO mice **(****FIGS. 1A** and **1B****,** and **FIGS. 8A-8D****).** CPT-11 treatment induced p53 and its targets PUMA and p21 within 6 hours in the intestinal mucosa of WT but not p53 KO mice **(****FIG. 1C** and **FIG. 8E****).** Puma KO did not significantly affect the expression of p53 or p21 **(****FIG. 1C****).** BH3-only proteins BIM and NOXA were not induced by CPT-11 or affected by Puma KO **(****FIG. 8F****).** RNA in situ hybridization (ISH) confirmed that Puma mRNA was undetectable in the crypts of untreated WT mice and strongly induced in the CBCs and some +4 cells by CPT-11 treatment **(****FIG. 1D****).** CPT-11 causes dose-dependent lethal GI injury in mice. To determine the sensitivity of C57BL/6J mice, survival after three consecutive daily doses of CPT-11 was monitored. The 180, 215, and 250 mg/kg per day doses resulted in 30, 90, and 100% lethality, respectively, and all deaths occurred between days 5 and 8, characteristic of lethal GI injury **(****FIG. 9****).** At the 90% lethal dose (LD90) of WT mice (215 mg/kg per day for 3 days), all Puma KO mice survived for 30 days, whereas all p53 KO mice died between days 6 and 9 **(****FIG. 1E****).** Histological analysis confirmed lethal GI injury in WT and p53 KO mice on day 5, showing severe loss of crypts and shortening of villi, which was abrogated in Puma KO mice **(****FIG. 1F****).** Consistent with a dual role of p53 in intestinal recovery from radiation, our data demonstrate that selective inhibition of p53-dependent induction of PUMA and apoptosis, but not p21 or other functions, results in intestinal chemoprotection.

### Example 3. A small-molecule PUMAi protects against chemotherapy-induced crypt apoptosis and lethality

The inventors identified a number of potential PUMAis using a pharmacophore model of the key interactions of the PUMA BH3 domain with BCL-2 family proteins, in silico compound screening, and apoptosis assays. One of these compounds (PUMAi) was confirmed to inhibit interaction of PUMA/BCL-xL but not of BIM/MCL-1 **(****FIGS. 10A-10C****).** PUMAi did not reduce camptothecin (CPT)-induced apoptosis in colon cancer cell lines with different p53 or PUMA genotypes, including p53 WT (HCT 116), null (p53 KO), mutant (HT29, SW480, and DLD1), or PUMA null (PUMA KO) **(****FIG. 10D****).** PUMA was only induced by CPT treatment in p53 WT HCT116 cells, not in any p53-deficient line **(****FIG. 10E****).** The basal amount of PUMA was much higher in HCT 116 cells, compared to very low or undetectable expression in mouse intestinal mucosa **(****FIGS. 1C** and **1D****)** and in normal human small intestine and colon.

To examine the efficacy of PUMAi in vivo, WT mice was treated with vehicle or PUMAi (10 mg/kg, an empirical dose) 2 hours before a single dose of CPT-11 (200 mg/kg) and analyzed crypt apoptosis 6 and 24 hours after CPT-11 administration. PUMAi reduced TUNEL and active caspase-3 staining preferentially in the CBCs, compared to transit-amplifying (TA), +4 to 9, cells **(****FIGS. 2A** and **2B****,** and **FIGS. 11A** - **11D****).** PUMAi treatment did not further reduce crypt apoptosis in Puma KO mice **(****FIG. 11E****).** A highperformance liquid chromatography (HPLC) assay was then developed to assess PUMAi distribution in the intestinal mucosa and plasma at three time points, -1.5, +1, and +6 hours of CPT-11 administration (0 hour) **(****FIG. 11F****).** PUMAi concentration remained at 67 and 30% in the intestinal mucosa at +1 and +6 hours, respectively, compared to -1.5 hours, but dropped more quickly in the plasma **(****FIG. 2C****).** CPT-11 is rapidly cleared from the plasma, with a half-life around 36 min. The inventors found that PUMAi given 1 hour after CPT-11 treatment still reduced apoptosis effectively, suggesting that PUMAi does not affect CPT-11 conversion or intestinal absorption **(****FIG. 2D****).**

Further, PUMAi decreased CPT-11-induced lethality at three different doses **(****FIG. 2E** and **FIGS. 12A** and **12B).** At the LD90, PUMAi improved survival from 10 to 70%. Histological analysis confirmed reduced GI injury on day 5 in the PUMAi and Puma KO groups, compared to WT mice **(****FIGS. 1F** and **2F****).** PUMAi also increased the survival of mice after 9.5- and 15-gray total body irradiation **(****FIGS. 12C** and **12D****),** further supporting the idea that targeting p53-dependent apoptosis, but not other functions, selectively reduces treatment-induced normal tissue toxicity.

### Example 4. PUMA inhibition does not reduce antitumor activity of CPT-11

To determine whether PUMA inhibition provides selective intestinal chemoprotection, the inventors established subcutaneous Lewis lung carcinoma (LLC) tumors in immunocompetent WT and Puma KO mice. After tumors reached an average of 100 mm3 (day 11), mice received six doses of CPT-11 (200 mg/kg, intraperitoneally) over 2 weeks **(****FIG. 13A****).** Tumor engraftment and CPT-11 response were indistinguishable in WT and Puma KO hosts **(****FIG. 3A** and **FIG. 13B****).** In contrast, CPT-11-induced weight loss was reduced in Puma KO mice **(****FIG. 3B****),** correlated with preserved intestinal structure and barrier evident by higher villus height, crypt numbers, and lower neutrophil infiltration **(****FIGS. 3C-3G****).**

The effects of PUMAi were then determined in tumor-bearing mice. PUMAi (10 mg/kg) or vehicle was given 2 hours before and 20 hours after each dose of CPT-11. The PUMAi group showed comparable tumor response compared with the vehicle group **(****FIG. 4A** and **FIG. 13B****)** but was highly resistant to CPT-11-induced weight loss, intestinal damage **(****FIGS. 4B-4G****),** and lethality **(Table 3).** Furthermore, PUMAi or PUMA KO groups exhibited improved grooming and physical activity throughout the study based on daily inspections. LLC tumors in all three CPT-11-treated groups showed similar effects on proliferation or apoptosis **(****FIGS. 13C** and **13D****)** but lacked induction of p53, PUMA, or p21 **(****FIG. 13E****),** consistent with mutant p53. These studies demonstrate that PUMA inhibition attenuates CPT-11-induced enterotoxicity but not the establishment or therapeutic responses of p53 mutant tumors in immunocompetent hosts.

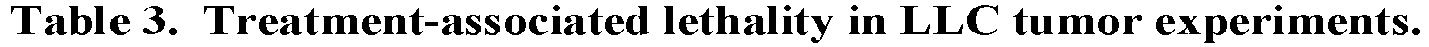

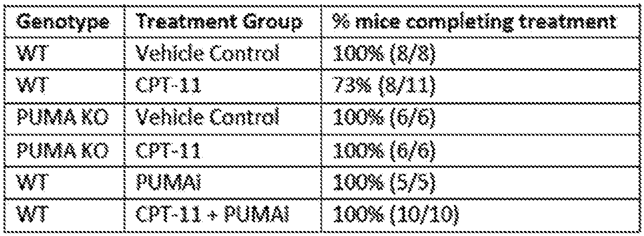

### Example 5. Targeting PUMA potently protects the LGR5⁺ stem cells and niche against chemotherapy

Little is known about the role of the ISC niche in injury-induced regeneration. The inventors tested whether the loss of CBCs, not of more chemosensitive TA cells, sends "injury" signals via niche "emptying" to activate remaining stem cells. PUMAi provides a pharmacological way to test this because it selectively blocks CPT-11-induced CBC apoptosis **(****FIG. 2B****).** Using LGR5-EGFP (enhanced green fluorescent protein) reporter mice, the inventors found that Puma KO or PUMAi decreases CPT-11-induced apoptosis of LGR5⁺ cells by more than 90 or 70%, respectively **(****FIG. 5A** and **5B****).** Puma KO also blocked LGR5⁺ cell apoptosis induced by another commonly used chemotherapy drug, 5-FU **(****FIG. 14****).** CD166 is a WNT target that marks the ISC niche, including CBCs and Paneth cells at the crypt bottom, in homeostasis. CPT-11 treatment induced a marked expansion of CD166+ cells from 48 to 96 hours in WT mice, which was fully suppressed by PUMAi treatment by 96 hours **(****FIGS. 5C** and **5D**). This was preceded by transient but strong activation of WNT (Lgr5, CD44, and Wnt3A)- and NOTCH (Olfm4, Math1, Hes1, Hes5, and Dll1)-responsive genes as early as 6 hours, which was largely suppressed in Puma KO and PUMAi-treated mice **(****FIG. 15****).** The only exception is the TA maker CD44, which was strongly suppressed by Puma KO but not by PUMAi **(****FIG. 5E****).** The inventors then monitored crypt DNA damage by 53BP1 foci, which was comparable in Puma KO, PUMAi, and control groups at 6 hours. However, DNA damage in Puma KO and PUMAi groups was lower by 48 hours after a single dose of CPT-11 and, more so, by 120 hours after three doses of CPT-11 **(****FIGS. 5F** and **5G****).** These data strongly suggest that the loss of LGR5⁺ cells disrupts the niche and triggers WNT and Notch signaling and intestinal regeneration after chemotherapy and that blocking LGR5⁺ cell loss likely delays compensatory proliferation and improves DNA repair.

### Example 6. LGR5⁺ cells are the critical target in intestinal chemoprotection by PUMAi

The inventors further tested that WNT and NOTCH activation triggered by LGR5⁺ cell loss will likely sensitize remaining stem cells to repeated exposures and stem cell exhaustion. After six doses of CPT-11 treatment, the LGR5⁺ crypt cells and the fraction of LGR5⁺ crypts were reduced by more than 90% in WT mice, compared to only 40 to 60% in the Puma KO and PUMAi groups **(****FIGS. 6A** and **6B****).** Olfm4 RNA ISH indicated a 93% drop in Olfm4+ crypts in WT mice, compared to 8 and 49% in the Puma KO and PUMAi groups, respectively **(****FIG. 6C****).** Paneth cells are the niche for LGR5⁺ cells and marked by MMP7 expression. Repeated CPT-11 treatment, but not single treatment, diminished MMP7+ cells within the crypts, with prominent mislocalization scattered through the epithelium and nearly complete loss of the close contact of MMP7+ and LGR5⁺ cells, which was suppressed in the Puma KO and PUMAi groups **(****FIGS. 6D** to **6F****).** Overall, these data demonstrate LGR5⁺ cell exhaustion as the key mechanism underlying chemotherapy-induced enterotoxicity, which can be effectively targeted by PUMAi.

### Example 7. PUMAi protects mouse and human colonic organoids against CPT-induced injury

To determine whether PUMAi acts directly on crypt cells, the inventors used a three-dimensional epithelial organoid or enteroid system previously used to demonstrate radiation-induced, PUMA-dependent LGR5⁺ cell apoptosis. CPT induced growth suppression and activation of caspase-3 in mouse colonic organoids, which was strongly blocked by PUMAi **(****FIGS. 7A** to **7C****).** PUMAi also suppressed CPT-induced activation of WNT and NOTCH targets (Lgr5, CD44, and Olfm4) **(****FIG. 7D****).** The suppression of TA marker CD44 was less pronounced compared to Lgr5 or Olfm4, similar to what the inventors observed in vivo.

To demonstrate the translational potential of PUMAi, the inventors used primary human colonic organoids. PUMAi enhanced organoid growth **(****FIGS. 7E** and **7F****)** and inhibited CPT-induced caspase-3 activation **(****FIG. 7G****).** PUMAi suppressed CPT-induced expression of WNT and NOTCH targets **(****FIG. 7H****),** establishing that the p53- and PUMA-dependent loss of LGR5⁺ cells triggers a substantial activation of ISC-related pathways within the epithelial compartment.

### Example 8. Additional compounds that inhibit PUMA-induced apoptosis.

In addition to PUMAi, several compounds were identified to demonstrate inhibition of PUMA-induced apoptosis using *in silico* screening of the ZINC 8.0 database, and *in silico* ADME/Toxicity profiles as described previously (Mustata G. et al. Curr Top Med Chem (2011) 11:281-290). The inventors also tested a group of compounds related to PUMAi for their ability to reduce PUMA-induced apoptosis in colon cancer cell line DLD1 and HCT-116. The structures and the apoptosis inhibition effects of these additional compounds are listed in the table below.

**Table 4. Structure and apoptosis inhibition of the compounds**

| ZINC code | Compound | Apoptosis inhibition of DLD1 | Apoptosis inhibition of HCT-116 |
|---|---|---|---|
| 19882186 | 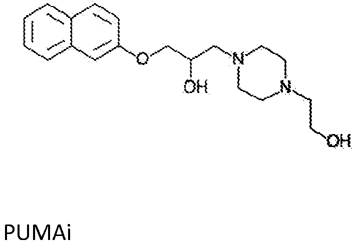 | 39% | 17% |
| 20647237 | 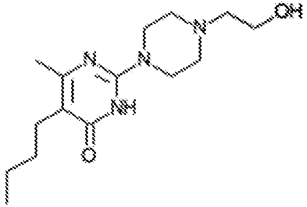 | 21% | Not determined |
| 20722356 | 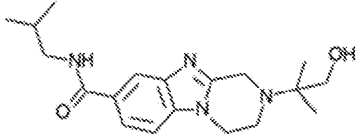 | 36% | Not determined |
| 21487182 | 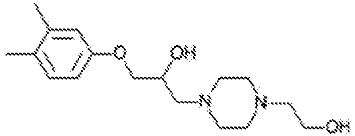 | 61% | 35% |
| 00062166 | 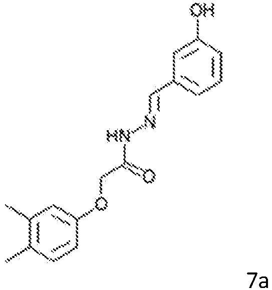 | 41% | 33% |
| 00060459 | 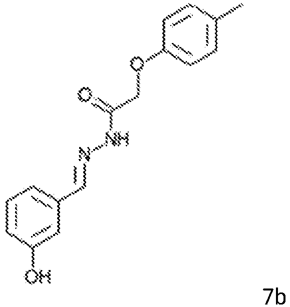 | -2% (no inhibition) | 21% |
| 00058956 | 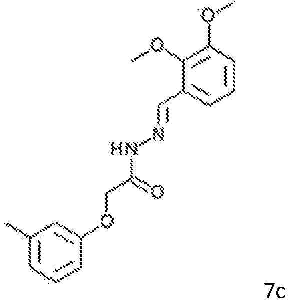 | 26% | 21% |
| 00062630 | 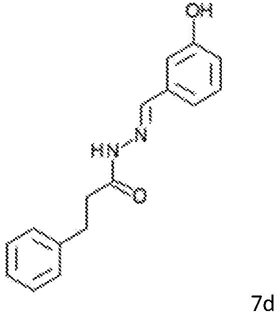 | 24% | 21% |
| 00048261 | 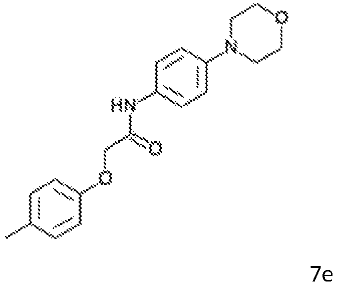 | 30% | 44% |
| 00068967 | 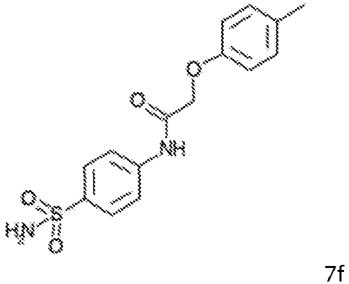 | -2% (no inhibition) | -8% (no inhibition) |
| 00034391 | 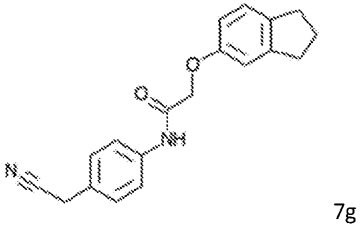 | 24% | -17% (no inhibition) |
| 00058831 | 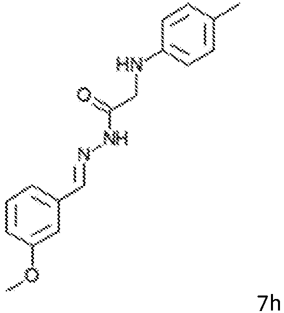 | 30% | 33% |
| 00059454 | 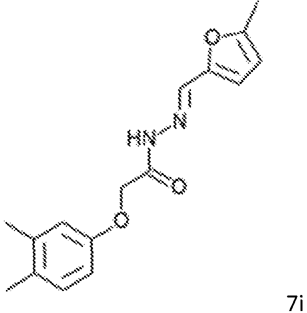 | 26% | 13% |
| 00059449 | 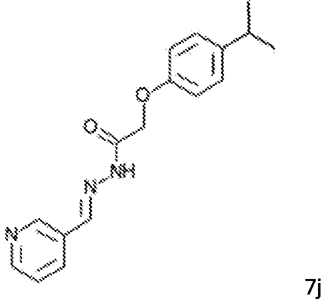 | 26% | -8% (no inhibition) |
| 00037563 | 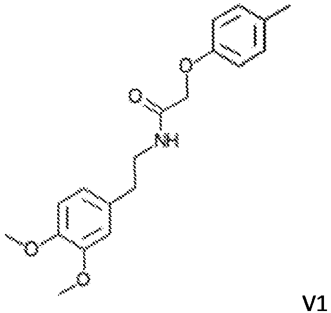 | 41% | 38% |
| 00035991 | 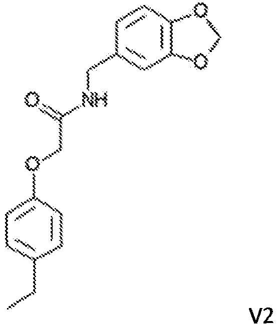 | 39% | 40% |

## Claims

1. A compound for use in a method for preventing or treating gastrointestinal injury induced by chemotherapy or radiotherapy, the method comprising:
administering to a subject a therapeutically effective amount of the compound, wherein the compound is 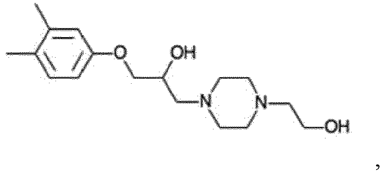 wherein the subject has received, is receiving or will receive a chemotherapy or radiotherapy.

2. The compound for use according to claim 1, wherein the subject has a cancer.

3. The compound for use according to claim 2, wherein the cancer is a cancer of the skin, breast, pancreas, prostate, ovary, kidney, esophagus, gastrointestinal tract, colon, brain, liver, lung, head and/or neck.

4. The compound for use according to claim 1, wherein administration of the compound inhibits activity of p53 upregulated modulator of apoptosis (PUMA) in at least a non-cancer cell, thereby preventing apoptosis of the non-cancer cell induced by the chemotherapy or radiotherapy, optionally wherein the non-cancer cell is a LGR5⁺ stem cell.

5. The compound for use according to claim 1, wherein administration of the compound does not protect cancer cells from the chemotherapy or radiotherapy.

6. The compound for use according to claim 1, wherein the chemotherapy is selected from the group consisting of irinotecan hydrochloride (CPT-11) and 5-fluorouracil (5-FU).

7. The compound for use according to claim 1, wherein the compound is deuterated.

## Patentansprüche

1. Verbindung zur Verwendung bei einem Verfahren zur Vorbeugung oder Behandlung einer durch Chemotherapie oder Strahlentherapie induzierten gastrointestinalen Verletzung, wobei das Verfahren Folgendes umfasst:
Verabreichen einer therapeutisch wirksamen Menge der Verbindung an ein Individuum, wobei es sich bei der Verbindung um 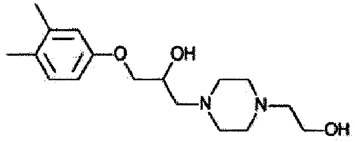 handelt,
wobei das Individuum eine Chemotherapie oder Strahlentherapie erhalten hat, erhält oder erhalten wird.

2. Verbindung zur Verwendung nach Anspruch 1, wobei das Individuum an einer Krebserkrankung leidet.

3. Verbindung zur Verwendung nach Anspruch 2, wobei es sich bei der Krebserkrankung um eine Krebserkrankung der Haut, der Brust, der Bauchspeicheldrüse, der Prostata, des Eierstocks, der Niere, der Speiseröhre, des Magen-Darm-Trakts, des Dickdarms, des Gehirns, der Leber, der Lunge, des Kopfes und/oder des Halses handelt.

4. Verbindung zur Verwendung nach Anspruch 1, wobei durch Verabreichung der Verbindung die Aktivität von p53-hochreguliertem Apoptosemodulator (PUMA) in mindestens einer Nicht-Krebszelle gehemmt wird, wodurch eine durch die Chemotherapie oder Strahlentherapie induzierte Apoptose der Nicht-Krebszelle verhindert wird, gegebenenfalls wobei es sich bei der Nicht-Krebszelle um eine LGR5⁺-Stammzelle handelt.

5. Verbindung zur Verwendung nach Anspruch 1, wobei durch Verabreichung der Verbindung Krebszellen nicht vor der Chemotherapie oder Strahlentherapie geschützt werden.

6. Verbindung zur Verwendung nach Anspruch 1, wobei die Chemotherapie aus der Gruppe bestehend aus Irinotecan-Hydrochlorid (CPT-11) und 5-Fluoruracil (5-FU) ausgewählt ist.

7. Verbindung zur Verwendung nach Anspruch 1, wobei die Verbindung deuteriert ist.

## Revendications

1. Composé pour utilisation dans un procédé de prévention ou de traitement d'une lésion gastro-intestinale induite par une chimiothérapie ou une radiothérapie, le procédé comprenant :
l'administration à un sujet d'une quantité thérapeutiquement efficace d'un composé, dans lequel le composé est 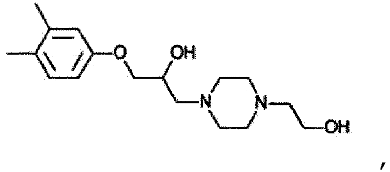
dans lequel le sujet a reçu, reçoit ou recevra une chimiothérapie ou une radiothérapie.

2. Composé pour utilisation selon la revendication 1, dans lequel le sujet a un cancer.

3. Composé pour utilisation selon la revendication 2, dans lequel le cancer est un cancer de la peau, du sein, du pancréas, de la prostate, de l'ovaire, du rein, de l'œsophage, du tube digestif, du côlon, du cerveau, du foie, du poumon, de la tête et/ou du cou.

4. Composé pour utilisation selon la revendication 1, dans lequel l'administration du composé inhibe l'activité du modulateur de l'apoptose régulé à la hausse par p53 (PUMA) dans au moins une cellule non cancéreuse, empêchant ainsi l'apoptose de la cellule non cancéreuse induite par la chimiothérapie ou radiothérapie, éventuellement dans lequel la cellule non cancéreuse est une cellule souche LGR5⁺.

5. Composé pour utilisation selon la revendication 1, dans lequel l'administration du composé ne protège pas les cellules cancéreuses de la chimiothérapie ou radiothérapie.

6. Composé pour utilisation selon la revendication 1, dans lequel la chimiothérapie est choisie dans le groupe constitué du chlorhydrate d'irinotécan (CPT-11) et du 5-fluorouracile (5-FU).

7. Composé pour utilisation selon la revendication 1, dans lequel le composé est deutéré.
